# EUROPEAN PATENT SPECIFICATION

(11) **EP 0 740 650 B1**
(45) Date of publication and mention of the grant of the patent: **26.05.2004**
(21) Application number: 94909643.2
(22) Date of filing: 17.02.1994
(51) Int. Cl.: C07C 229/00, C07C 62/06

(54) **CODRUGS AS A METHOD OF CONTROLLED DRUG DELIVERY**
KOMBINIERTE WIRKSTOFFE ALS METHODE ZUR GEZIELTEN WIRKSTOFF-FREISETZUNG
CO-MEDICAMENTS UTILISES COMME PROCEDE D'APPORT REGULE DE MEDICAMENTS

(30) Priority: 28.01.1994 US 187462
(43) Date of publication of application: 06.11.1996
(73) Proprietor: UNIVERSITY OF KENTUCKY RESEARCH FOUNDATION, Lexington, KY 40506-0059 (US)
(72) Inventor: ASHTON, Paul, Lexington, KY 40515 (US); CROOKS, Peter, Anthony, Lexington, KY 40502 (US); RIGGS, Robert, Mack, Lexington, KY 40514 (US); CYNKOWSKI, Tadeusz, Lexington, KY 40508 (US); CYNKOWSKA, Grazyna, Lexington, KY 40508 (US); HONG, Guo, Lexington, KY 40503 (US)
(74) Representative: Chapman, Paul Gilmour
(86) International application number: PCT/US1994/001659
(87) International publication number: WO 1995/020567

(56) References cited:
- EP-A- 0 392 745
- EP-A- 0 484 870
- GB-A- 2 278 843
- US-A- 5 157 151
- ALBRECHT H A ET AL: "CEPHALOSPORIN 3'-QUINOLONE ESTERS WITH A DUAL MODE OF ACTION1" JOURNAL OF MEDICINAL CHEMISTRY, vol. 33, no. 1, 1 January 1990 (1990-01-01), pages 77-86, XP000257048
- HONG ET AL.: "Nucleoside Conjugates V. Synthesis and Biological Activity of 9-beta-D-Arabinofuranosyl)adenine Conjugates of Corticosteroids" JOURNAL OF PHARMACEUTICAL SCIENCES, vol. 73, no. 2, 1984, pages 278-280, XP002063289
- HASHIDA ET AL.: "Characterization of a Lipophilic Prodrug of 5-Fluorouracil with a Cholesterol Promoiety and Its Application to Liposomes" CHEM. PHARM. BULL., vol. 36, 1988, pages 3186-3189, XP002063290
- LIN ET AL.: "(o-and p-Nitrobenzyloxycarbonyl)-5-fluorouracil Derivatives as Potential Conjugated Bioreductive Alkylating Agents" J. MED. CHEM., vol. 29, no. 1, 1986, pages 84-89, XP002063291
- SEI-ICHI NISHIMOTO ET AL: "1-(5'-FLUORO-6'-HYDROXY-5',6'-DIHYDROURAC IL-5'-YL)-5-FLUOROURACIL, A NOVEL N(1)-C(5)-LINKED DIMER THAT RELEASES 5-FLUOROURACIL BY RADIATION ACTIVATION UNDER HYPOXIC CONDITIONS" JOURNAL OF MEDICINAL CHEMISTRY, vol. 35, no. 14, 10 July 1992 (1992-07-10), page 2711/2712 XP002019528
- DOUBLE ET AL.: "Nucleoside analogues. 5. Molecular combination of anti-cancer/nitrosurea combinations against mouse colon tumours" ANTI-CANCER DRUG DESIGN, vol. 1, no. 2, 1986, pages 133-140, XP002063292
- KINGSBURY ET AL.: "A Novel Peptide Delivery System Involving Peptidase Activated Prodrugs as Antimicrobial Agents. Synthesis and Biological Activity of Peptidyl Derivatives of 5-Fluorouracil" J. MED. CHEM., vol. 27, no. 11, 1984, pages 1447-1451, XP002063293

## Description

### Technical Field

The present invention related to the field of controlled pharmaceutical delivery, especially to codrug compounds.

### Background Art

A prodrug is a compound formed by chemical modification of a biologically active compound which will liberate the active compound in vivo by enzymatic or hydrolytic cleavage. The primary purpose of employing a prodrug for oral administration is to increase intestinal absorption or site specific absorption or to reduce local side effects, such as gastrointestinal irritation. Prodrugs may also be used to increase transdermal absorption, by enhancing permeation through topical membranes.

On this basis, prodrugs are not generally classified as sustained release dosage forms. However, the ability to bioreversibly modify the physicochemical properties of a drug allows better intestinal transport properties and hence can influence the drug blood levels versus time profile of the pharmaceutical compound. Thus, prodrugs can be used to increase the strategies for sustained release and, in a limited sense, can be sustaining in their own right.

United States Patent No. 5,176,907 to Leong et al. discloses biocompatible and biodegradable poly(phosphoester-urethanes). The patent describes therapeutic agent delivery vehicles which include polymers which are biodegradable because of the hydrolyzable phosphoester or P-(O)-O-C bond. A particular aspect of the Leong patent is a therapeutic agent that can be introduced into the poly-phosphoester urethane by covalently binding a radical of this therapeutic agent to the phosphorous atom of the polymer. The patent describes attaching 5-fluorouracil to polyurethane. The patent discloses that drugs with carboxyl groups can be coupled to the phosphorous atom via an ester bond which is hydrolyzable.

United States Patent Number 5,194,581 to Leong et al. discloses biodegradable polyphosphoesters. A therapeutic agent is pendently bound to a poly(phosphoester) polymeric matrix. When the therapeutic agent is pendently attached, it is chemically linked through, for example, ionic or covalent bonding. The drug is released when the polymeric agent biodegrades. A combination of one or more therapeutic agents can be incorporated into the composition of the invention. The patent discloses therapeutic agents containing two hydroxyl groups that can be directly incorporated into the backbone of the polymers. Other therapeutic agents can be derivatized for incorporation into the backbone. For instance, a drug with two amino groups can be reacted with the carboxyl group of a hydroxyl carboxylic acid. The hydroxyl groups can then be used to form the poly(phosphoester). A sustained delivery is effected by the hydrolysis of the polymeric prodrug. Although Leong discloses that two therapeutic agents may be bound to a polymer matrix via covalent bonding, it does not disclose or suggest that two therapeutic agents can be linked to one another by covalent bonding as a prodrug as in the present invention.

United States Patent No. 5,104,877 to Boger discloses a psoriasis treatment. Boger describes a carboxy-protecting group used as a prodrug where the carboxy-protecting group can be readily cleaved in vivo. These carboxy groups are indicated to be used in the protection of carboxyl groups in penicillin and cephalosporin.

United States Patent No. 4,489,065 to Walton et al., discloses chondroitin drug complexes. The '065 patent describes that the rate of drug release can be controlled in a variety of ways, such as by encapsulation in a material which dissolves slowly in the body fluids, by entrapment in a bolus or matrix from which the drug diffuses slowly, or by conversion into a so called "prodrug", in which the drug is bound with another substance turning it into a substantially inactive compound or complex. The drug is gradually released by physiological action when injected into the tissues of the patient. The '065 patent discloses chondroitin or chondroitin sulphate covalently or ionically bonded to a drug substance of the group consisting of chloramphenicol, methotrexate, adriamycin, vinblastine, vincristine, vindesine, 6-mercaptopurine, 5-fluorouracil, penicillin antibiotics, cephalosporin antibiotics, and oxacephalosporin antibiotics, to form a prodrug. The patent states that the prodrug provides controlled release of the drug in a physiological environment. The patent discloses that a variety of functional groups are available in chondroitan for covalently bonding (particularly carboxyl, COOH, and hydroxyl, OH) and for ionic bonding (sulfate -OSO₃-, and carboxylate, -COO-) with drugs. Covalent bonding can be by way of ester links, -COOY, or amide links, -CONHY-. When chondroitin and the drug substance contain a hydroxyl and an amino group, the reaction can proceed through the formation of a carbamate bond via activation of the hydroxyl to a chloroformate moiety with subsequent linking to the amine function. The rate of release of the drug from the chondroitin or from the linking substance is dependent on the type of bonds chosen for linkage.

United States Patent No. 5,130,126 to Koyama et al. discloses a polymer-drug conjugate and a method of producing it. Polymers which may be used have an alkyleneoxy group as a repeating unit such as polyoxyalkylene glycol as well as polymers obtained by substituting the terminal groups of the polymers with an acyl, amino or allyl group. Polymers are combined with drugs using covalent bonding, ionic bonding, coordinate bonding, and shift base formation.

United States Patent No. 5,057,301 to Wilbur et al. discloses modified cellular substrates used as linkers for increased cell retention of diagnostic and therapeutic agents. The invention of Wilbur et al. comprises a ligand-linker conjugate wherein the linker is a chemically modified cellular substrate having a protein conjugation group attached thereto. The protein conjugation group attached to the modified substrate linker is a functional group which will react with the group on the targeting protein and form a bond between the linker and the protein. Suitable protein conjugation groups include active esters (including carboxylic esters, imide esters, succinimidyl esters, phenolic esters, and imidate esters), primary or secondary amines, hydrazides, hydrazines, carboxylate, isothiocyanates, isocyanates, and Michael-type acceptor groups such as maleimides, thiols, anhydrides and alkyl halides.

United States Patent No. 5,171,566 to Mizushima et al. discloses a flurbiprofen derivative ophthalmic preparation. The derivative is an ester of flurbiprofen. United States Patent No. 4,933,324 to Shashoua is directed to a fatty acid-neuroactive drug conjugate used as a prodrug and involves the formation of a prodrug from a fatty acid carrier and a neuroactive drug. The bond between the fatty acid and the drug may be an amide or an ester bond. United States Patent No. 4,975,278 to Senter et al. discloses antibody-enzyme conjugates in combination with prodrugs for the delivery of cytotoxic agents to tumor cells.

United States Patent No. 4,267,326 to Ozaki et al. discloses uracil derivatives. The uracil derivatives are prepared by reacting 5-fluorouracil with an α-haloalkyl carboxylate or an aldehyde diacylate.

United States Patent No. 4,897,260 to Ross et al. discloses glucocorticoid carboxylic acid esters which include triamcinolone acetonide 21-oic methylester for the treatment of xeroderma pigmentosum.

United States Patent No. 4,910,192 to Avery et al. discloses topically active steroidal anti-inflammatory agents. The agents are 12-β substituted glucocorticoids wherein the 12 substituent is a hydroxyl group or a lipophilic group attached to a 12-β-hydroxyl group. The lipophilic group may be selected from an alkyl or aryl substituted ester, an ester, a carbamate and a carbonate groups. The 12th substituent can be a lower carboxylic acid ester of a 12-β-hydroxy group.

United States Patent No. 5,177,064 to Bodor discloses targeted drug delivery via phosphonate derivatives. United States Patent No. 5,112,835 to Miyasaka et al. discloses 6-substituted acyclopyrimidine nucleoside derivatives for use as antiviral agents. Chemical Abstracts, volume 117(8), abstract no. 76315 (m) discloses the stereoselective enzymatic hydrolysis of various ester prodrugs of ibuprofen and flurbiprofen.

Chemical Abstracts, Volume 116(18), abstract no. 181046 (b) describes the preparation of prodrugs of flurbiprofen, its 1,2-ethanediol ester and 1,4-butanediol ester. The prodrugs showed high stability in simulated gastric fluid, simulated intestinal fluid and simulated pancreatic fluid. The drugs showed less toxicity and increased anti-inflammatory and analgesic effects. Journal of Medicinal Chemistry, vol.33, (1990), 77-86 teaches cephalosporin 3'-quinolone esters having a broadened antibacterial spectrum. Journal of pharmaceutical sciences, vol. 73, (1984), 278-280; Chemical of Pharmaceutical Bulletin, vol. 36, (1988), 3186-3189; EP-A-0 484 870; Journal of Medicinal Chemistry, vol.29, no.1, 1986, pages 84-89; Journal of Medicinal Chemistry, vol.35, (1992), 2711-2712; Anti-cancer Drug Design, vol.1, no.2, 1986, pages 133-140; EP-A-0 392 745; GB-A-2 278 843; Journal of Medicinal Chemistry, vol.27, (1984), 1447-1451 disclose codrugs comprising anti-cancer, or anti-neoplastic, or anti-tumoral drug.

None of the above patents disclose or suggest prodrug conjugates of two or more of the same or different drugs linked to one another. Nor do they disclose codrug conjugates which are linked by reversible covalent bonds, such as ester, carbamate and carbonate bonds, so that at the desired site in the body they are cleaved to regenerate the active forms of each of the drugs.

Patient compliance in consumption of pharmaceutical compositions as part of a therapeutic regimen is critical for patient recovery and treatment. This is especially critical in elderly patients which may have poor memory and which exhibit poor patient compliance with a therapeutic regimen of doses of pharmaceutical compositions. Other high risk compliance groups include drug addicts, alcoholics and those requiring long term therapy, for example tuberculosis patients.

Furthermore, known erodible, implantable pharmaceutical substances such as polylactic acid and polyurethane compounds are formulated such that it is difficult to achieve high drug loading and hence difficult to deliver large doses of drug from the substrate. Such drug substances have low solubility.

There is a need in the pharmaceutical arts for pharmaceutical compounds which deliver two or more drugs at a single time in a single dose, which exhibit controlled drug delivery. In one embodiment, the pharmaceutical compounds of the present invention are delivered in a totally erodible drug delivery device capable of delivering two or more synergistic drugs over a prolonged period. The codrug compounds of the present invention have the advantage that linking the two drug compounds decreases the solubility of each through the carbamate, carbonate and ester bonds linking the compounds. The codrug compounds of the invention have a high degree of chemical or enzymatic lability at physiological pH 7.4.

Albrecht et al (J. Med. Chem. 1990, 33, 77 - 86) discloses antibacterial quinolones linked to a cephalosporin by an ester bond through the cephalosporin 3'-position.

Hong et al (J. Pharmaceutical Science, 1984, 73(2), 278 280) discloses 1-(β-D-arabinofuranosyl) adenosine (vidarabine) conjugates of corticosteroids with increased water solubility.

Hashida et al (Chem. Pharm. Bull. 1988, 36(8),3186 - 3198) relates to the generation of a lipophilic prodrug of 5-fluorouracil (5FU) with a cholesteryl promoiety in order to entrap this within a liposome.

EP484870 discloses an antibody-β-lactamase conjugate and a cephalosporin - antitumour agent prodrug which is designed to be targeted to bind to tumour cells and the antitumour agent released on action of the β-lactamase.

Lin et al (J. Med. Chem. 1986, 29, 84 - 89) relates to conjugates of alkylating (anti-cancer) agents and 5-fluorouracil (5-FU).

Nishimoto et al (J. Med. Chem. 1992, 35, 2711 - 2712) describes dimmers of 5-fluorouracil which are intended to be activated by radiation.

Double et al (Anti-cancer drug design, 1986, 1, 111 - 123) teaches combinations of 5-fluorouracil and nitrosoureas for treatment of colon cancer.

EP392745 discloses a drug delivery system in which an immunoconjugates and a prodrug are used in association with one another to deliver a drug to a host target site.

### Brief Description of the Figures

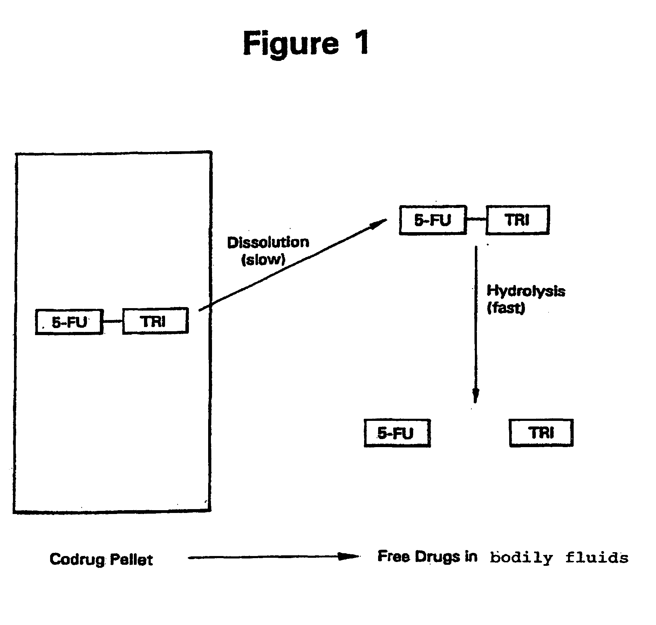

Figure 1 shows a typical release mechanism. Amongst the advantages of these systems are that as no polymers are required to control release so the devices can be extremely small (small enough to be fitted onto a haptic of an intraocular lens).
Figure 2 shows devices were implanted subconjunctivally in each eye of ten rabbits.
Figure 3 shows codrug devices are entirely composed of drug and do not require release rate controlling polymers enabling extremely small systems to be prepared. 1.5 mm pellets containing 1.5 mg of TRI and 0.5 mg 5FU were small enough to be fixed to the haptics of IOLs.
Figure 4 shows bioerodible codrug implants were prepared 1.5 mm in diameter and attached to 6-0 nylon suture. Implants were immersed in 5 ml of phosphate buffer (pH 7.4) and samples periodically removed for HPLC assay to determine the release of both 5-fluorouracio (5FU) and triamcinolone acetonide (TRI). Implants were then inserted into the vitreous of 14 New Zealand White rabbits.
Figure 5 shows that codrug formulations may be used in the treatment of arthritic conditions by injection into the affected joint.

### Disclosure of the Invention

An object of the invention provides sustained release delivery of two or more pharmacologically active compounds. The drug compounds may be the same or different.

Also provided for is a codrug composition, comprising at least two drug compounds covalently linked to one another via a labile bond to form a single codrug composition.

Also provided for is a codrug composition wherein drug compounds may be linked by labile bonds to another entity such as polyethylene glycol, glycerol or a sugar.

An object of the invention provides a codrug wherein at least one or more of the drug compounds is selected from the group consisting of an antiviral compound, a beta-blocker, an antibacterial compound, and a biological compound with pharmacological activity.

The invention provides a codrug composition in solid form, a codrug composition which is applied topically for examples in a form selected from the group consisting of a transdermal patch, ointment, cream, suspension, liquid and eyedrop.

A codrug according to the invention may be administered by a method selected from the group consisting of injection, inhalation, implantation, applied nasally such as a nasal spray, applied rectally, ingested orally and applied vaginally.

Another embodiment of the invention provides a codrug composition attached to a surgically implantable device, for example, a coding attached to a suture. Another embodiment of the invention provides a codrug composition is in the form of a nonerodible delivery vehicle which for example comprises polyvinyl alcohol.

The codrug of the invention may comprise from 0.1 to up to about 100% of said nonerodible delivery vehicle.

Another object of the invention is to provide local delivery of two or more synergistic pharmacologic agents or delivery of two or more non-synergistic pharmacologic, agents. Another object of the invention provides 5FU linked to two molecules of FB, while another object provides 3α,17α-21-trihydroxy 5β pregnane-20-one.(THS) linked to 5FU and FB.

The totally erodible drug delivery device is capable of delivering two or more synergistic drugs over a prolonged period.

Another object of the invention is to control the release rate of 5FU and triamcinolone from pellets of 5FU/TRI in a buffer.

Still another object of the invention is directed to the use of codrugs in the inhibition of posterior capsular opacification (PCO) after extracapsular cataract extraction and intraocular lens implantation.

A further object of the invention investigates the feasibility of codrug technology as a means to achieve the intravitreal delivery of 3α,17α-21-trihydroxy 5β pregnane-20-one. (THS), a model angiogenesis inhibiting steroid, and 5FU.

Another object of the invention provides a totally bio-erodible sustained release system for 5FU and triamcinolone in the eye.

### Description of the Invention

In a first aspect the present invention provides a bioerodible composition that provides sustained release of active drugs in a bodily fluid, comprising a codrug having at least two active drugs covalently linked to one another via a labile bond, wherein the codrug has low solubility in bodily fluids, such that the codrug undergoes slow dissolution into the bodily fluid followed by rapid hydrolysis of the labile bond, thereby regenerating the active drugs.

In a further aspect the present invention provides use of a codrug having at least two active drugs covalently linked to one another via a labile bond for the manufacture of a bioerodible medicament for providing sustained release of active drugs in a bodily fluid, wherein the codrug has low solubility in bodily fluids, such that the codrug undergoes slow dissolution into the bodily fluid followed by rapid hydrolysis of the labile bond, thereby generating the active drugs.

The present invention provides a means of improving the pharmaceutical and pharmacological properties of pharmacologically active compounds or prodrugs by conjugating them together to form a codrug.

Codrugs are formed by conjugation of two or more agents via a labile linkage. Codrug conjugates may be linked via reversible covalent bonds such as ester, carbonate, cyclic phosphate ester and carbamate bonds, so that the required site in the body they are cleaved to regenerate the active forms of the drug compounds. Bonds may be, but are not limited to the type 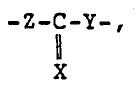 wherein Z is O, N, CH2O or CH2S, Y is O, S or N, and X is O or S. The rate of cleavage of the two drugs can be controlled by the type of bond, the choice of drugs and the physical form of the conjugate. The bond selected may be enzyme specific. The bond may be selected from enzymatically labile bonds, for example, to esterases as in the ACV-FB linkage, or may be chemically labile (eg. base catalyzed hydrolysis of the 5FU-TRI linkage). The codrugs are labile in water, serum or other bodily fluids and regenerate the active parent drugs. The present invention is the first to combine two or more drugs in the form of a codrug which generates two active drug compounds with improved pharmaceutical properties.

In an embodiment of the present invention, codrugs have the applicability of providing a controlled or sustained release for a systemic or local pharmacologic or physiologic effect relating to the following areas: treatment of cancerous primary tumors; chronic pain; tuberculosis; arthritis; rheumatic conditions; hormonal deficiencies such as diabetes; and modifications of the immune response such as in the prevention of transplant rejection and in cancer therapy. A wide variety of disease states may be prevented or treated using the codrug compositions of the present invention. Such disease states are known to those of ordinary skill in the art (see Goodman and Gilman, The Pharmacological Basis of Therapeutics, 8th Ed., Pergamon Press, NY, 1990; and The Merck Index, 11th Ed., Merck and Co., Inc., Rahway, NJ 1989.

In addition, codrug compositions of the present invention are suitable for treating mammalian organisms infected with the AIDS, manifestations of AIDS such as Kaposi's sarcoma, and AIDS related opportunistic infections such as cytomegalovirus retinitis, toxoplasmosis, Pneumocystis carnii and microbacterial avium intracellular.

A codrug of the invention may consist of one or more pharmacologically active compounds in the following classes of agents; anesthetics and pain killing agents such as lidocaine and related compounds and benzodiazepain and related compounds; anticancer agents such as 5-fluorouracil, adriamycin and related compounds; anti-inflammatory agents such as 6-mannose phosphate; anti-fungal agents such as fluconazole and related compounds; antiviral compounds such as trisodium phophomonoformate, trifluorothymidine, acyclovir, ganciclovir, dideoxyinosine (ddI), dideoxycytidine (ddC) and acyclovir; cell transport/mobility impeding agents such as colchicine, vincristine, cytochalsian B and related compounds; anti-glaucoma drugs such as carbonic anhydrase inhibitors, beta blockers, miotics, cholinesterase inhibitors, and sympathomimetics; immunological response modifiers such as muramyl dipeptide and related compounds; cytokines and peptides/proteins such as cyclosporin, insulin, growth factor or growth hormones and steroids. Non steriodal anti-inflammatory agents include, for example, flurbiprofen and indomethacin.

Codrugs may also be formed of unstable drugs and other compounds to improve their stability such as levodopa and the peripheral decarboxylase inhibitor benserazide.

Codrug formulations may comprise a number of other substituents to optimize release, bioavailability or appearance and may be used in sustained release devices or systems. Such substituents are known to those of ordinary skill in the art and for example are set forth in Remington's Pharmaceutical Sciences, 18th Ed., Mack Publishing Co., Easton, PA, 1990.

Another embodiment of the present invention comprises a codrug compound in a nonerodible matrix or reservoir system containing natural or synthetic polymers that are biologically compatible with and essentially insoluble in body fluids. Such materials include for example, but are not limited to polyvinyl acetate, polyvinyl alcohol, cross-linked polyvinyl butyrate, ethylene ethyl acrylate copolymer, polyethyl hexyl acrylate, polyvinyl chloride, polyvinyl acetals, plasticized ethylene vinyl acetate copolymer, ethylene vinyl chloride copolymer, polyvinyl esters, polyvinyl butyrate, polyvinyl formal, polyamides, polymethylmethacrylate, poly butyl methacrylate, plasticized polyvinyl chloride, plasticized nylon, plasticized soft nylon, plasticized polyethylene terethphalate, natural rubber, polyisoprene, polyisobutylene, polybutadiene, polyethylene, polytetrafluoroethylene, polyvinylidine, chloride, polyacrylonitrile, cross-linked polyvinyl pyrrolidone, polytrifluorochloroethylene, chlorinated polyethylene, poly(1,4,-isopropylidne diphenylene carbonate), vinylidine chloride, acrylonitrile copolymer, vinyl chloride-diethyl fumarate copolymer, silicone rubbers ( especially medical grade polydimethylsiloxanes, ethylene-propylene rubber, silicone-carbonate copolymers, vinylidine chloride-vinyl chloride copolymer, vinyl chloride-acrylonitrile copolymer and vinylidine chloride acrylonitrile copolymer.

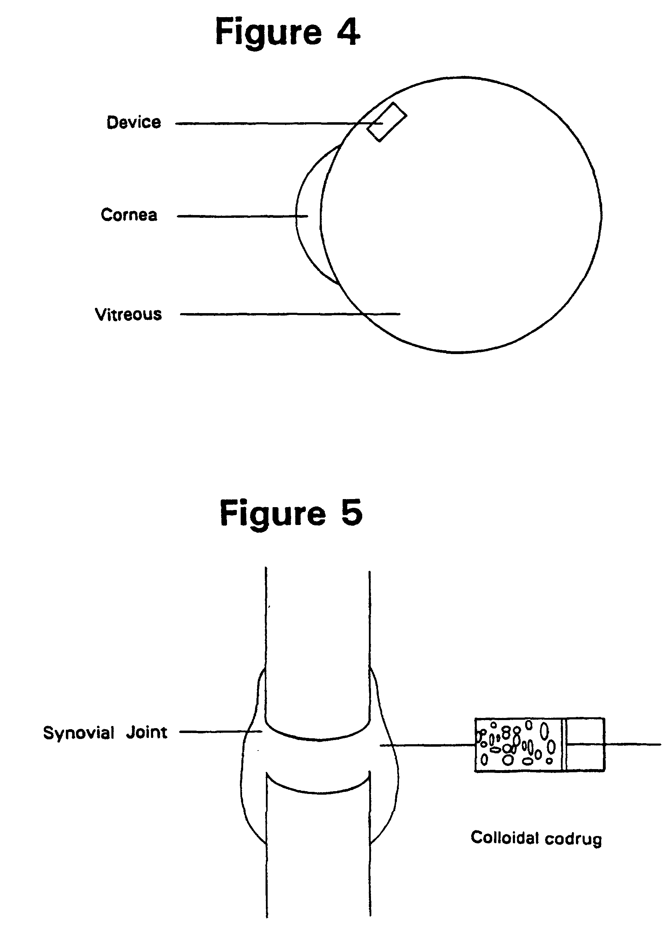

In another embodiment, a totally bioerodible sustained release system for pharmacologically active agents is composed of codrug alone (either solid, liquid or colloidal). Injectable codrug systems have a variety of applications including, but not limited to arthritis (figure 5).

The codrug of the invention may be administered in injectable form selected from the group consisting of liposomes, liquids, suspensions and microsphere nanoparticles. Preparation of such aqueous solutions, liposomes, emulsion and suspensions are known to those of ordinary skill in the art (see Remington' s Pharmaceutical Sciences, 18th Ed., Mack Publishing Co., Easton, PA, 1990, pp. 1504-1712, incorporated herein by reference).

Another embodiment of the invention provides a totally bioerodible sustained release system for pharmacologically active agents composed of codrug in a formulation with another bioerodible substance such as polyvinyl acid, polyanyhydride, collagen, or polyalkylcyanoacrylates such as polybutycyanoacrylate.

Examples of codrugs of the present invention include 5-fluorouracil with corticosteroids, acyclovir with flurbiprofen and timolol (a beta-blocker) with the prostaglandin PGF2 alpha. These codrugs are labile when dissolved in bodily fluids and are rapidly hydrolyzed to regenerate the two active parent drugs. In the solid form however, they are stable, even in an aqueous environment because in order to hydrolyze they must first be in solution.

Pellets of codrugs of the invention, therefore, slowly release drugs in solution or bodily fluids reflecting the low solubility of the conjugated forms. Pellets may be formulated from the codrug compounds alone or with implantable, bioerodible substances may be selected from polylactic acid and polyglycolic compounds. Pellets may be formulated by methods known in the art and may contain 0.1 to about 100% of the codrug composition.

Codrugs may also be formulated in bioerodible or nonbioerodible delivery systems to further control their release. Such bioerodible systems include polylactic acid (bioerodible) to form a film around, or a matrix with a codrug to further improve the pharmaceutical properties. Polylactic acid can be formulated in solutions of 2, 5 and 10% polylactic acid, and has been used to produce 5FU-TRI codrug pellets attached to sutures. 2% polyvinyl alcohol has been used to coat pellets of 5FU-THS for subconjunctival delivery. Polybutyl cyanoacrylate (bioerodible) has been used to form a matrix with 5FU-TRI pellets attached to an intraocular lens haptic and silicone (nonbioerodible) to attach the same pellets to lens haptic (see Example 7 below).

Furthermore, in one embodiment of the invention a pharmacologically active composition possessing some undesirable effects may be conjugated to another agent to reduce the undesirable effects such as isoniazid with pyroxidine. Another embodiment of the invention is a codrug formulated with other drug or prodrug molecules.

Amongst the advantages of codrug systems are that frequently no polymers are required to control release so that the devices can be extremely small (small enough to be fitted onto a haptic of an intraocular lens). Codrugs systems can also be formulated as suspensions (nanoparticle size range) and upper size limitations are only imposed the application method under consideration. There are also no concerns of residual polymer after drug has been released, nor of polymer related toxicity as no polymers are used in the construction of the devices.

Some specific examples of codrugs of the invention are given below:

### Example 1 Codrug from triamcinolone acetonide and bis(hydroxymethyl)-1-5-fluorouracil (See scheme 1 below)

Bis(hydroxylmethyl)-5-fluorouracil (2) (158 mg) was dissolved in 5ml. of acetonitrile in an ice bath. To this stirred solution 112 µL of triethylamine was added followed by triamcinolone acetonide 21-chloroformate (1) prepared from 240 mg of triamcinolone acetonide. The resulting solution was stirred at room temperature overnight, concentrated in vacuo, redissolved in methylene chloride and washed with water and brine. The crude product was chromatographed on silicagel using chloroform - methanol = 100:5 as a solvent system, 210 mg of solid codrug was obtained. Yield 61.4% ¹H-NMR (CDCl₃); 0.95 (s,3H C-18), 1.2 (s,3H C-19), 1.4, 1.55 (2s,6H isopropylidene), 3.25 (m, 1H C-16), 4.4 (m, 1H C-11), 4.8 5.15 (2d, 2H C-21), 5.0 (d, 1H OH), 5.7-5.85 (2d, 2H CH₂N), 6.15 (s,1H C-4), 6.35 (d,1H C-2), 7.3 (d,1H C-1), 7.65 (d,1H), 10.0 (s,1H).

### Example 2 Hydrolysis of 5FU/TRI

This example measured the chemical and enzymatic hydrolysis of the 5FU/TRI codrug and to determine the release of drug entities.

A stock solution was prepared by dissolving 10 mg of 5FU/TRI in 10 ml acetonitrile. This was then added to a series of phosphate buffers at pH 3, 5, 6.4, 7.4 and 8.4 at 37°C to give final concentrations of 100 ug/ml. Care was taken to ensure that these solutions were indeed solutions and not suspensions. Samples were periodically removed and assayed by HPLC as described below. Enzymatic hydrolysis was determine in a similar way using pooled serum from 3 volunteers. The assay procedure used distinguished between TRI and 5FU/TRI. 5FU was assayed under different conditions.
HPLC Assays: Samples of buffer containing codrug and steroids were assayed by HPLC using a fully automated Hitachi system with a C-18 reverse phase column (25 cm × 4 mm x 5 µm) and uv detection. The mobile phase was 40% acetonitrile buffered to 4.0 with 0.02% sodium acetate. The flow rate was 1.0 ml/min and detection was at 238 nm. Under these conditions the retention time of the codrug 5FU/TRI was 17 minutes while triamcinolone acetonide eluted at 9 minutes. Quantitation limits were 0.3 and 0.5 ug/ml, respectively. Under the above conditions 5FU was found to elute with the solvent front and so was assayed separately. For 5FU an Applied Biosystems HPLC system was used with a C-18 column reverse phase column (25 cm x 4 mm x 5 µm) and 0.02% sodium acetate buffer mobile phase (Ph 4.0). The flow rate was 1.0 ml/min and detection was by uv at 266 nm. Under these condition the retention time was 6.5 minutes and the detection limit 0.2 ug/ml.

Samples of serum were deproteinated before assay by HPLC. 300 µl serum samples were added to 300 µl acetonitrile in a microcentrifuge tube. After vortex mixing for 10 seconds tubes were centrifuged at 14,000 rpm for 30 minutes. To determine the concentration of codrug and steroid the supernatant was injected directly onto the HPLC (sensitivity to 0.6 ug/ml and 1.0 ug/ml, respectively).

To quantitate 5FU it was necessary to remove acetonitrile before analysis. 300 µl of the supernatant was dried under reduced pressure using a speed vacuum. The dried plug was then rehydrated with 150 µl deionized water before assay by HPLC. Interference from serum residues reduced sensitivity to 1 ug/ml.

The codrug was hydrolyzed in a first order process to quantitatively generate 5FU and triamcinolone acetonide. The rate of hydrolysis was much faster at high pH and was extremely fast in serum (half-life, t_{½}, less than 10 minutes).

| pH | t_{½} |
|---|---|
| 3.0 | 204 hr |
| 5.0 | 12.7 hr |
| 6.4 | 78 min |
| 7.4 | 14.1 min |
| 8.4 | 9.8 min |
| Serum | 8.8 min |

This examples shows that although the 5FU/TRI codrug is stable in acidic conditions, it is highly labile under physiological conditions, breaking down to regenerate 5FU and triamcinolone acetonide.

### Example 3: 5FU/TRI as a Sustained Release System In Vitro

This example measures the release rate of SFU and triamcinolone from pellets of 5FU/TRI in phosphate buffer.

Two mg pellets of the codrug 5FU/TRI were prepared in a 1.5 mm pellet press using a modified Parr Instrument Press. Pellets were then immersed in 5 ml phosphate buffer (Ph 7.4, 37 °C) and 300 µl samples removed each day for 6 days; there immediately replaced was replaced 300 µl of buffer. Samples were assayed by HPLC for 5FU/TRI, triamcinolone acetonide and 5FU. After 6 days buffer was completely replaced, to maintain sink conditions, and sampling continued.

No intact codrug was detected in the receptor medium by HPLC (detection limit 0.5 ug/ml). Release of triamcinolone acetonide was found to follow pseudo zero order kinetics with a mean release rate of 1.4 +/- 0.3 ug/hr. Release of 5FU was found to be 0.4 +/- 0.06 ug/hr.

These rates were maintained until over 60% of triamcinolone acetonide and 5FU had been released. the mean ratio of 5FU to triamcinolone acetonide concentration (ug/ml) in the receptor solution was 3.40 +/- 0.15 at all time points, (equimolar release).

This example demonstrates that codrug delivery systems can be used as sustained release agents however a more thorough *in vivo* evaluation must be performed.

### Example 4 5FU/TRI as a Sustained Release System In Vivo

This example measures the release rate and vitreous concentrations of 5FU and TRI from pellets of 5FU/TRI in the rabbit vitreous.

Two mg pellets of the codrug 5FU/TRI were prepared using a 1.5 mm press. These pellets were then fixed onto 6-0 nylon suture using intraocular lens grade silicone. silicone covered the base of the pellets providing a platform for suture attachment. Alternatively a solution of 2% or 5% polylactic acid was used. Pellets attached by both methods were implanted into the vitreous of 8 New Zealand white rabbits through a small incision through the sclera. For each type of device, two animals were killed after 1, 2, 3, and 4 weeks and the vitreous and aqueous obtained from the frozen sphere. Before death animals were examined with a slit lamp. This platform did not effect release rate *(in vitro).* Pellets were then implanted into the vitreous of 8 New Zealand White rabbits in a similar manner to that described in 3b except that sclerotomy sites were smaller in this case (2.5 mm). Two animals were sacrificed (You may want to change this word) after 1, 2, 3 and 4 weeks and vitreous and aqueous obtained from the frozen sphere. HPLC analysis was then performed on tissue samples and explanted devices to determine the concentrations of 5FU, triamcinolone acetonide and intact 5FU/TRI.

No intact codrug was detected in the vitreous. Mean intravitreal levels of triamcinolone were found to be 3.0 +/- 0.9 ug/ml. These levels were maintained for three weeks, declining to 0.8 +/- 0.4 ug/ml on the fourth week before dropping below the detection limit of the HPLC by week 5.

This experiment shows that sustained levels of TRI and 5FU in the vitreous are achievable by the implantation of a codrug device. Such a device has the advantages of being composed entirely of the required drugs and of being completely bioerodible. No toxic or inflammatory effects are anticipated as the only compounds being released are 5FU and triamcinolone acetonide.

### Example 5 5FU-TRI Codrug Implant to Control Scar Formation in Strabismus Surgery

An in vivo evaluation of scar reduction under 5-fluorouracil (5FU)-triamcinolone (TRI) implant after extraocular muscle (EOM) surgery in rabbits, an experimental animal model for correlation to human utility.

In each eye of ten rabbits the inferior rectus muscle (IRM) was disinserted from the globe and electrocautery was used to create a scar between the muscle and sclera. After the IRM was sewn back into its insertion site, four 2 mg codrug implants were inserted between sclera and muscle in the right eye, while the left was used as a control. Bioerodible codrug implants releasing 5FU and TRI over 6 weeks were prepared. Devices release equimolar amounds of 5FU and TRI. Animals were sacrificed at 1, 2 and 3 weeks and eyes eviscerated. Specimens were prepared and stained with H&E. The scar thickness and cellular infiltrate were determined quantitatively using a Bioscan image analysis program.

Drug treated eyes showed an 80% decrease in scar thickness compared to controls (7 to 35 µm). Microscopic examination of a defined area of tissue (40 µm²) revealed a greater number of inflammatory cells present in the control tissue (> 300 versus < 35). The codrug implant may prove useful in scar reduction following EOM surgery.

### Example 6 Bioerodible Sustained Release Subconjunctival Co-Delivery of TRI and 5FU

This example determined feasibility of a codrug release system for the subconjunctival co-delivery of 5FU and TRI for possible use in glaucoma filtration surgery.

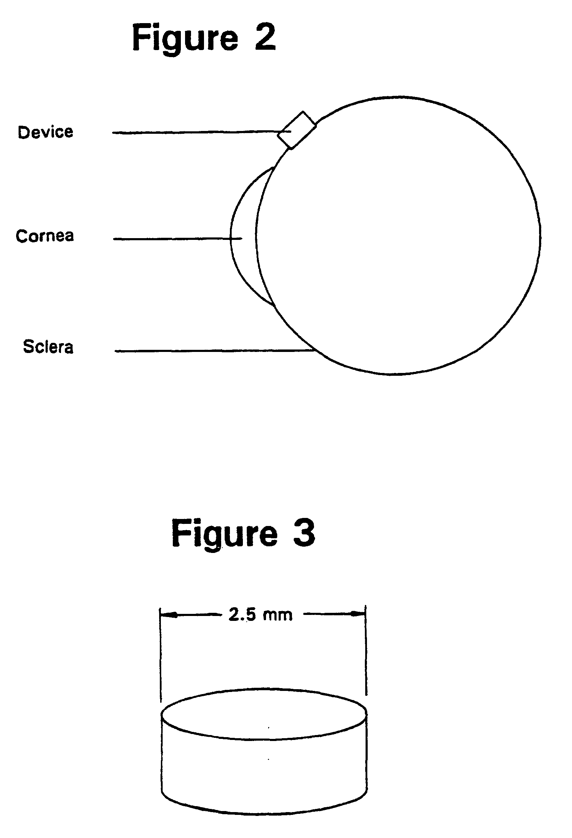

Codrug devices were prepared as flat discs 2.5 mm in diameter weighing 5 mg. Each device contained approximately 3.7 mg of TRI and 1.3 mg of 5FU and were composed of over 97% active substance. Devices were implanted subconjunctivally in each eye of ten rabbits (figure 2). Toxicity and inflammation were determined by weekly slit lamp examinations and electroretinograms (ERGs). Animals were sacrificed after 3, 7, 10 and 14 days and eyes eviscerated. After freezing at -70°C devices were removed for determination of residual drugs and the complete vitreous and aqueous dissected from the ice ball to determine the concentration of SFU and TRI. Two animals were used for histology and were sacrificed 6 weeks after implantation.

Analysis of explanted devices showed 5FU and TRI to have been released at a pseudo zero order rate of 9 +/-1% per day over the first ten days. Devices released equimolar amounts of 5FU and TRI. ERGs were normal for all animals and there was no evidence of toxicity or inflammation around the implantation site.

### Example 7 Codrugs in the Prevention of Posterior Capsular Opacification

This example investigates the use of codrugs in the inhibition of posterior capsular opacification (PCO) after extracapsular cataract extraction and intraocular lens implantation in the rabbit.

Codrug pellets were prepared that gave pseudo zero order release of 5FU and TRI in an equimolar ratio over 6 weeks. Codrug devices are entirely composed of drug and do not require release rate controlling polymers enabling extremely small systems to be prepared. 1.5 mm pellets containing 1.5 mg of TRI and 0.5 mg 5FU were prepared and fixed to the haptics of IOLs using the bioerodible polybutyl cyanoacrylate. This polymer is soaked into the pellet forming a codrug/cyanoacrylate matrix as it dried (figure 3). 16 New Zealand White rabbits were used in this study, one eye (control) received a polymethyl methacrylate intraocular lens (IOL) (Chiron Intraoptics) while the other received an IOL with codrug. Eyes were examined regularly by slit lamp and PCO scored from 0 to 4+. Retinal function was determined by electroretinogram (ERG) before implantation and before sacrifice. Animals were sacrificed after 4, 8, 12 and 16 weeks; eyes were removed and fixed in formalin. Photographs of posterior capsule were projected onto a grid and the percentage of PCO calculated.

ERG and histopathologic data indicated that the codrugs were well tolerated with no indication of a toxic or inflammatory response. Slit lamp examination showed a statistically significant decrease in PCO between study and control eyes (p< 0.001). The less subjective assessment of PCO using a grid confirmed this observation and indicated an arrest of opacification in drug eyes with a statistical significance of p< 0.03 by 8 weeks.

This example indicates that codrug implants are well tolerated in the capsular bag. Significantly, the development of PCO can be controlled over a prolonged period by the use of these implants.

### Example 8 Intravitreal Co-Delivery of TRI and 5FU

This example evaluates intravitreal co-delivery of 5FU and TRI in an animal model of proliferative vitreoretinopathy (PVR). Previous studies indicate that TRI and 5FU is useful in the treatment of PVR.

Bioerodible codrug implants were prepared 1.5 mm in diameter and attached to 6-0 nylon suture. Implants were immersed in 5 ml of phosphate buffer (pH 7.4) and samples periodically removed for HPLC assay to determine the release of both 5FU and TRI. Similar implants were then inserted into the vitreous of 14 New Zealand White rabbits (figure 4). Toxicity was assess by electroretinogram and slit lamp examination in all animals. Histopathologic examination was performed on four animals. Ten animals were used for pharmacokinetics; two animals were sacrificed 1, 2, 3, 4 and 5 weeks after implantation. Eyes were enucleated, devices removed and both vitreous and aqueous obtained. All samples were assayed by HPLC. Five animals received actual devices in one eye and placebo implants in contralateral eyes. These were sacrificed after 3 and 6 weeks for histopathologic examination.

Devices released TRI at 1.4 ug/hr and 5FU at 0.3 µg/hr in buffer (equimolar release) and were well tolerated in rabbit eyes with no indication of toxicity or inflammation. Vitreous levels of TRI were maintained at 2.4 µg/ml over the 5 weeks duration of the pharmacokinetic study.

The delivery system described gives pseudo zero order release of both TRI and 5FU in buffer. As the devices are small, they can be readily inserted into a normal scleral MVR blade incision. The devices appear to be well tolerated and maintain high, potentially therapeutic, drug levels in the vitreous. Levels of TRI and SFU in the aqueous were too low to be detected. Future work will evaluate the use of this codrug system in a PVR model.

### Example 9 Intravitreal Delivery of an Anti-Neovascular Agent and an Antiproliferative Agent in the Rabbit Eye

The example shows codrug technology as a means to achieve the intravitreal delivery of 3α, 17α, 21-trihydroxy 5β pregnane-20-one (THS), a model angiogenesis inhibiting steroid, and 5FU. THS has no corticosteroid activity but inhibits neovacularization in the chick embryo and rabbit cornea models. Activity of this and related agents can be enhanced by a variety of agents including aurin tricarboxylic acid, and cyclodextran.

Other workers have reported that antimetabolites can also inhibit neovascularization. Increased efficacy of THS may be anticipated from the coadministration of 5FU. we have developed a bioerodible implantable device by preparing a 5FU/THS codrug. In vitro, devices release 2 moles of 5FU for each mole of THS.

2 mg codrug devices were implanted into the vitreous of 20 New Zealand White rabbit eyes (ten animals) through 2.5 mm incisions parallel to and 3 mm from the limbus. Animals were also examined by slit lamp and retinal function was assessed by electroretinogram (ERG) examination before implantation and immediately before sacrifice. Animals were periodically sacrificed after implantation and eyes immediately enucleated and frozen. Vitreous was then assayed for 5FU, THS and intact codrug by HPLC. Explanted devices were also assayed for residual drug.

### Proliferative vitreoretinopathy

To treat proliferative disorders in the vitreous or lens capsule such as proliferative vitreoretinopathy or posterior capsular opacification therapeutic concentrations of 5FU (over 0.5 ug/ml) and corticosteroid (1 ug/ml) should be maintained so as to at the same time inhibit fibroblast proliferation (SFU inhibits fibroblasts) and prevent inflammation which stimulates their proliferation (TRI has potent anti-inflammatory properties).

Method to achieve sustained release of two or more pharmacologically active compounds or codrugs from an injectable formulation. A 10 mg/ml suspension of 5FU-TRI. codrug was prepared in isotonic phosphate buffer. This was injected into the vitreous of 3 rabbits. One animal was killed after 1, 3 and 7 days and both eyes were removed. HPLC analysis was performed on each eye to detect intact codrug, TRI and 5FU. Injection of the suspension was found to maintain therapeutic levels of both 5FU and TRI. in the vitreous over the 7 day duration of this study.

### Example 10

A 5FU/TRI codrug conjugate according to the invention was found to be unstable in buffer at pH 7.4 although stable in pH 3.0 (respective t½ less than 3 minutes and over 2 days).

The compound is moreover relatively insoluble in bodily fluid so that a pellet can be compressed that does not readily dissolve in buffer at pH 7.4, but slowly releases both TRI and 5FU over an extended period of time (months) even when immersed in pH 7.4. The advantage of such a system is that although each of the parent compounds are released, the intact conjugate is never detected in solution (its half-life is too short). This conjugate is can be formulated into a totally bio-erodible sustained release system for 5FU and triamcinolone in the eye. Both agents are presently used in combination and a sustained release form for one or the other has been a goal of ophthalmologists for a long period.

In similar way a conjugate of 5FU and TRI can be used as a codrug compound for the treatment of proliferative vitreoretinopathy (PVR). A pellet of such an implant with similar properties to the above is implanted intravitreally after vitrectomy and is found to reduce the occurrence of PVR. Animal studies are proceeding.

An additional manifestation of the codrug idea is the conjugation of an anti-viral compound (ganciclovir or acyclovir) with a nonsteroidal anti-inflammatory agent (flurbiprofen or indomethacin). These conjugates are insoluble and would be suitable for subconjunctival implantation in herpes keratitis.

### Example 11

The following is the structure of 5FU linked via a carbonate bond to a glycerol diflurbiprofen ester. The rationale is that the compound would hydrolyze *in vivo* to release 5FU, glycerol and two molecules of flurbiprofen.

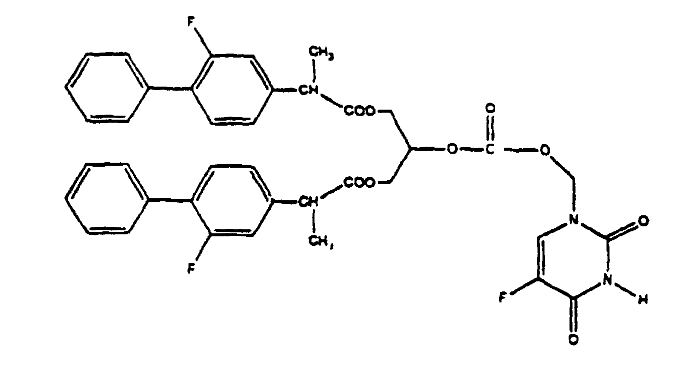

### Example 12 Codrug from flurbiprofen and acyclovir ( See scheme 2 below)

200mg of acyclovir, 160 mg of flurbiprofen, 200 mg of dicyclohexylcarbodiimide (DCC) and 13 mg of dimethylamiknopyridine (DMAP) were mixed with 7 ml of dimethylformamide. The mixture was stirred at 55°C overnight, then evaporated to dryness under vacuum. The solid residue was chromatographed on silicagel to yield 340 mg of the codrug (3d). ¹H-NMR (DMSO), 1.4 (d, 2H CH₂O), 3.8 (q, 1H CH), 4.1 (m,2H), 5.3 (s,2H NCH₂O), 6.5 (s, 2H NH₂), 7.15-7.55 (m,8H arom.), 7.8 (s, 1H CH).

### Example 13 Codrugs from bis(hydroxymethyl)-5-fluorouracil and flurbiprofen (See scheme 5 below)

Flurbiprofen acid chloride (282 mg) was dissolved in 3 ml of acetonitrile. To this stirred solution triethylamine (142 mg) was added followed by 5-fluorouracil (2) derivative (170 mg). The cloudy mixture was stirred at room temperature overnight, diluted with dichloromethane, then washed with water and brine. Chromatography on silica gel yielded 2 codrugs (4a). Yield 145 mg of monosubstituted product and 160 mg of bissubstituted product. ¹H-NMR (acetone) for monoester, 1.7 (d,3H CH₃), 3.9 (q, 1H CH), 5.75 (s,2H CH₂N), 7.2-7.6 (m,8H arom.), 7.92 (d,1H). ¹H-NMR (acetone) for diester, 1.5 (m, 6H 2CH₃), 3.75 (m, 2H 2CH), 5.6 (s,2H CH₂N), 6.0 (s, 2H CH₂N), 7.0-7.6 (m,16H arom.).

### Example 14 Codrug from prostaglandin PG_{2α}and timolol

102 mg of protected prostaglandin PG_{2α} was dissolved in 4.5 ml of methylene chloride at 0°C. To this solution carbonyldiimidazole (35 mg) was added and the resulting solution was stirred at 0°C for 40 min. The solution of timolol (54 mg) in 1 ml of methylene chloride was then added and the mixture was heated at 50-54°C overnight. The solution was washed with water and brine. Thus obtained crude product was purified by chromatography and redissolved in tetrahydrofuran (3 ml) at 0°C. To this stirred solution tetrabutylammonium fluoride was added. After 0.5 h the solvent was evaporated, the residue was dissolved in ethyl acetate, washed with dil. sodium bicarbonate solution, brine and dried over Na₂SO₄. The oily residue was purified by preparative TLC to yield the expected codrug (39% of yield). ¹H-NMR (CDCl₃), 0.85 (t, 3H CH₃), 1.1 (s, 9H t-Bu), 2.56 (d,2H CH₂N), 3.5 (m,4H), 3.8 (m,4H), 3.9-4.2 (m,4H 3CHO+OH), 4.6 (m, 2H CH₂O), 5.25 (m, 1H CH-O), 5.3-5.6 (m, 4H olefine).

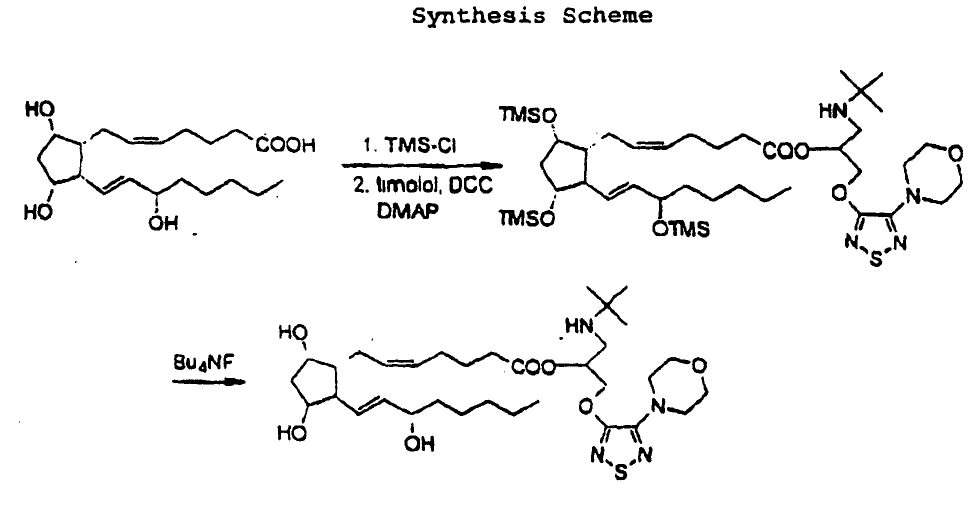

### Example 15 Codrug made of 5β-pregnane-3α,17α,21-triol-20-one, flurbiprofen and 5-fluorouracil (scheme 4)

1.4 ml of the solution of phosgene in toluene and 1 ml of the THF were cooled to 0°C in an ice-bath. To this stirred mixture the solution of (5) (60 mg) and triethylamine (14.5 µL) in 1.5 ml of THF was slowly added. After 6 h the excess of phosgene and the solvent were removed in a stream of nitrogen. The residue was diluted with 1 ml of acetonitrile and the solution of bis(hydroxymethyl) -5-fluorouracil (2) (50 mg) and triethylamine (29 µL) in 1.5 ml of acetonitrile was added. The resulting homogenous solution was kept in refrigerator overnight. The residue obtained after solvent evaporation was purified by preparative TLC yielding 52 mg of the codrug (6). ¹H-NMR (CDCl₃), 0.6 (s, 3H C-18), 0.9 (s,3H C19), 1.52 (d,3H CH₃), 3.7 (q,1H CH), 4.75 (m,1H C-3), 4.8-5.3 (2d,2H C-21), 5.7 (s,2H CH₂N), 7.1-7.55 (m,8H arom.), 7.6 (d, 1H CH).

### Example 16

Esters of acyclovir with flurbiprofen and indomethacin have been synthesized as shown in Scheme 2 set forth below, from the corresponding acids (activated with N,N-dicyclohexylcarbodiimide). Using this method the ester of flurbiprofen **3a** has been readily obtained, but when indomethacin was used the amidoester **3b** surprisingly was isolated from the reaction mixture.

The conjugate of ganciclovir and indomethacin has been synthesized as presented in Scheme 5. This diester could not be obtained by simple esterification. However, when the primary amino group was protected as a N-trityl derivative **7** (via the intermediate diactetate), the acylation with excess of indomethacin acid chloride gave the expected diester **8**.

Synthesis of the monoester of ganciclovir with flurbiprofen, Scheme 6, required selective protection of one of the two primary hydroxyl groups in ganciclovir **9**. This was achieved by treating the latter with 2.5 eq. of monomethoxytrityl chloride in the presence of triethylamine and DMAP. The resulting ditrityl derivative **10** was treated with flurbiprofen acid chloride to give the fully protected monoester **11.** Removal of the trityl groups with acetic acid provided the desired codrug **12**.

### Example 17

### Conjugates of 5-fluorouracil with flurbiprofen, indomethacin, and triamcinolone acetonide.

5FU remains a clinically important antiviral and antitumor agent, but it possesses high toxicity and far from optimal delivery properties. Synthesis of a series of 5FU conjugates with anti-inflammatory drugs such as flurbiprofen, indomethacin, and triamcinolone acetonide resulted in compositions with improved properties.

5FU can be attached to hydroxy compounds as carbamate (via the intermediate chloroformate) as shown in Scheme 7 below.

In the case of menthol **13**, the stable product has been obtained. If, however, an oxygen atom is introduced at the proximity of the hydroxyl group, the carbamate bond becomes very labile. Via NMR the inventors were able to prove a carbamate bond was formed. An attempt to prepare the carbamate from 5FU and triamcinolone acetonide-21-chloroformate 1 failed completely. The inventors investigated the esterification of the known 1,3,-bis-(hydroxy-methyl)-5FU **2** with acid chlorides and chloroformated. Compound 2 has been obtained from 5FU and formalin as viscous oil containing ca. 60% of the bis-(hydroxymethyl) derivative and ca. 35% of both the isomeric mono (hydroxymethyl) products. This mixture was used in all subsequent reactions without further purification.

Flurbiprofen and indomethacin acid chlorides were coupled with **2** in acetonitrile in the presence of triethylamine to give a mixture of the mono and diesters (Scheme 3).

In both cases the major product was the 1-substituted derivative and the separation of the mixture did not present any difficulty.

An alternative approach involved the use of hydroxyesters of flurbiprofen to attach the 1,3-bis-(hydroxymethyl)-5FU via a carbonate link (Schemes 8 and 9).

In the scheme 8 example, the monoester of flurbiprofen and triethylene glycol **13** was prepared. the synthesis required selective protection of one of the two hydroxyl groups as a silyl derivative. Subsequent acylation and deprotection led to the expected monoalcohol. This product was chloroformylated with THF wit a solution of the phosgene and coupled with **2** to yield the desired codrug **14**.

Alternatively, flurbiprofen 1,3-diglyceride **15** was obtained by the methods of Scheme 9. In the first approach, dihydroxyacetone was readily acylated with flurbiprofen acid chloride in the presence of pyridine, and the central keto group was rapidly reduced by sodium borohydride in THF solution. A purification procedure of column chromatography on silica gel led to the expected monoalcohol **14.**

The second approach involved the preparation of 1,3-benzylidene glycerol, protection of the remaining hydroxyl group as an O-benzyl derivative, acidic hydrolysis of the acetal and acylation of the diol with flurbiprofen acid chloride. Finally the benzyl group was removed by transfer hydrogenation in the presence of 10% Pd/C. Compound **15** was chloroformylated and coupled with the 5-fluorouracil derivative as described above, to yield the desired codrug **16**.

Triamcinolone acetonide was bonded to the 1,3,-bis-(hydroxymethyl)-5FU **2** via a carbonate (Scheme 1) linkage. The product, obtained after the treatment of TRI with phosgene, contained only one chlorformyl group. Steric considerations make it practically impossible for the reaction to occur with the 11β-hydroxyl group. The monochloroformate obtained above was coupled with **2** to give the expected crystalline codrug **17** after chromatographic purification.

### Example 18

Codrugs based on 5β-pregnane-3α ,17α,21-triol-20-one.

Triple codrugs composed of 3 components including an antimetabolite agent (5FU), and anti-inflammatory agent ( flurbiprofen) and an antivasculating agent (5β-pregnane-3α,17α,21-triol-20-one).

Initially, two model compounds **19** and **20** (Schemes 10 and 11) were synthesized and evaluated with respect to the stability in aqueous solution as a function of pH.

The easily available 5β-androgen-3α-ol-17-one (18) was acylated with flurbiprofen acid chloride in pyridine in the presence of DMAP. The resulting ketoester **19** was then reduced in high yield to the alcohol **20.** For the completion of the synthesis, the alcohol was chloroformylated in the usual fashion, and coupled with 5FU to yield the expected product **21.**

In the second synthesis model, the simple ester **22** of flurbiprofen and 5β-pregnane-3α,17α,21-triol-3,20-dione was obtained as shown in Scheme 11. Synthesis of all "triple" codrugs have been based on the easily available and relatively cheap Reichstein's Substance 23 (Scheme 4). This material was transformed in the usual way (formalin and concentrated HCL in methylene chloride) its bismethylenedioxy derivative, which was then hydrogenated in high yield with palladium-on-calcium carbonate in the presence of potassium hydroxide, to the saturated 5β-pregnanone. This ketone was then reduced with sodium borohydride, predominantly to the equatorial alcohol **24**; the axial alcohol was obtained as a minor side product. The acylation of **24** with flurbiprofen acid chloride yielded the ester **25.** For the completion of the synthesis, the dihydroxyacetone side chain was liberated by treatment of **25** with hydrofluoric acid in THF, and the resulting diol **5** was chloroformylated and coupled with **2** to give the desired codrug 6.

The synthesis of the next series of codrugs is shown in Scheme 12. The hydroxyl group of the alcohol **24** was protected as an O-benzyl derivative and then the bismethylenedioxy group was hydrolyzed in the usual fashion. The selective acylation of the 21-hydroxy group led to the flurbiprofen ester **26.** The benzyl group was removed by transfer hydrogenation and the resulting alcohol was converted to the chloroformate **27**. This product was then subject to coupling with either 1, 3-bis-(hydroxymethyl)-5FU 2 or 5FU itself, which gave the corresponding codrugs **28** and **29**, respectively. Independently the two codrugs containing only 5-Fu and 5β-pregnane-3α,17α,21-triol-20-one were prepared (Scheme 13).

The alcohol **24** was hydrolyzed and chloroformylated to the bis-chloroformate **30.** When compound **30** was coupled with a 3 equivalents excess of **2**, the expected codrug **31**, containing two 5FU residues, was obtained. However, if only a 1.5 equivalents excess of **2** was used, the monocarbonate **32** was isolated. The structure of **32** was proved by ¹H and ¹³C NMR analysis.

Due to the remarkable stability of flurbiprofen esters at pH 7.4, an alternative type of linear unit between flurbiprofen and the steroidal alcohols was considered (Scheme 14).

In the reaction of chloroformate **33** with the salt of flurbiprofen, it was expected that a mixed anhydride of flurbiprofen and carbonic acid would be obtained. Instead, however, the ester **34** was isolated as the only product. Obviously, **34** is formed from the unstable, intermediate mixed anhydride by elimination of carbon dioxide.

### Example 19 Derivatives of acetazolamide

Acetazolamide is a useful drug for the treatment of glaucoma. However, due to the unfavorable lipophilicity it is not active when given topically to the eye. Approaches to solve the delivery problems may include the development of appropriate codrug forms. Preparation of sulfocarbamate derivatives of acetazolamide are shown in Scheme 15. The product **35** appears to stable in buffer solution to ensure a sufficient rate and extent of codrug conversion to the parent drug at this time.

The synthesis schemes referred to above, appear below. 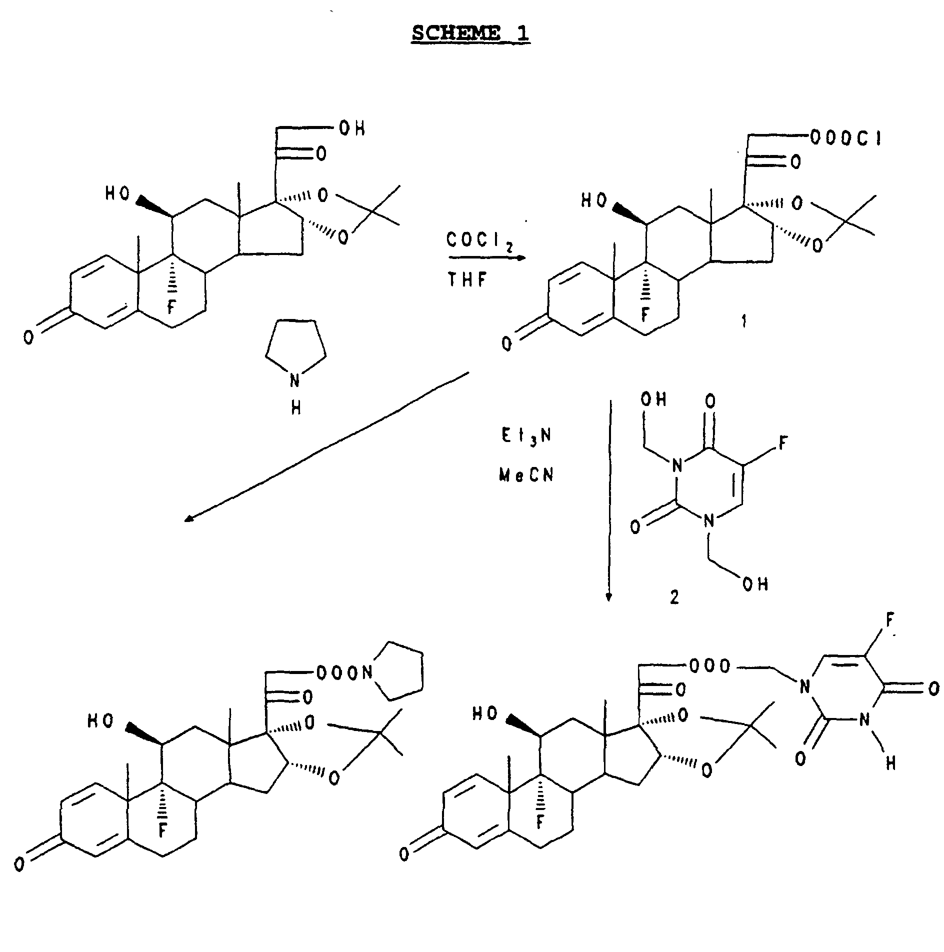 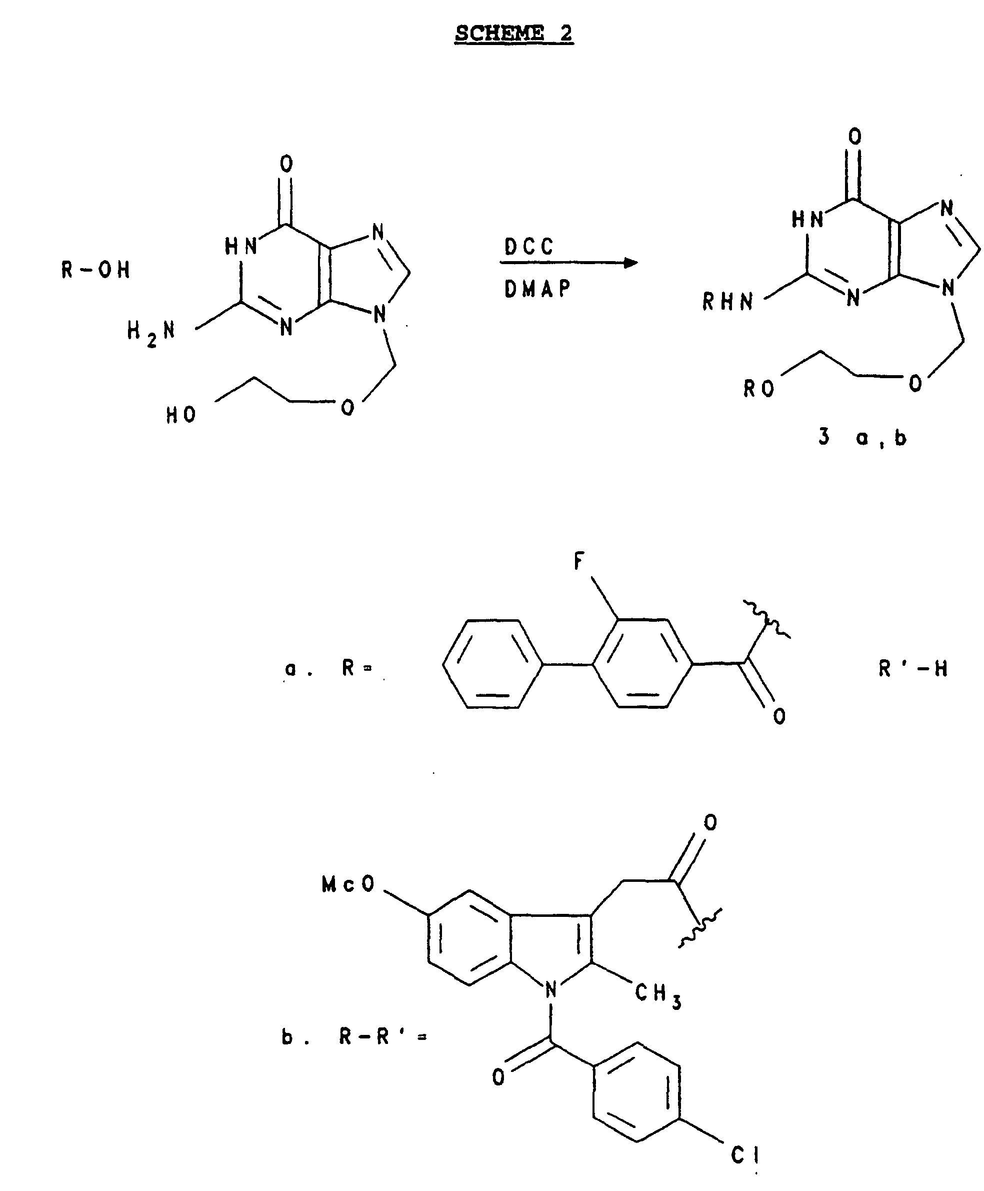 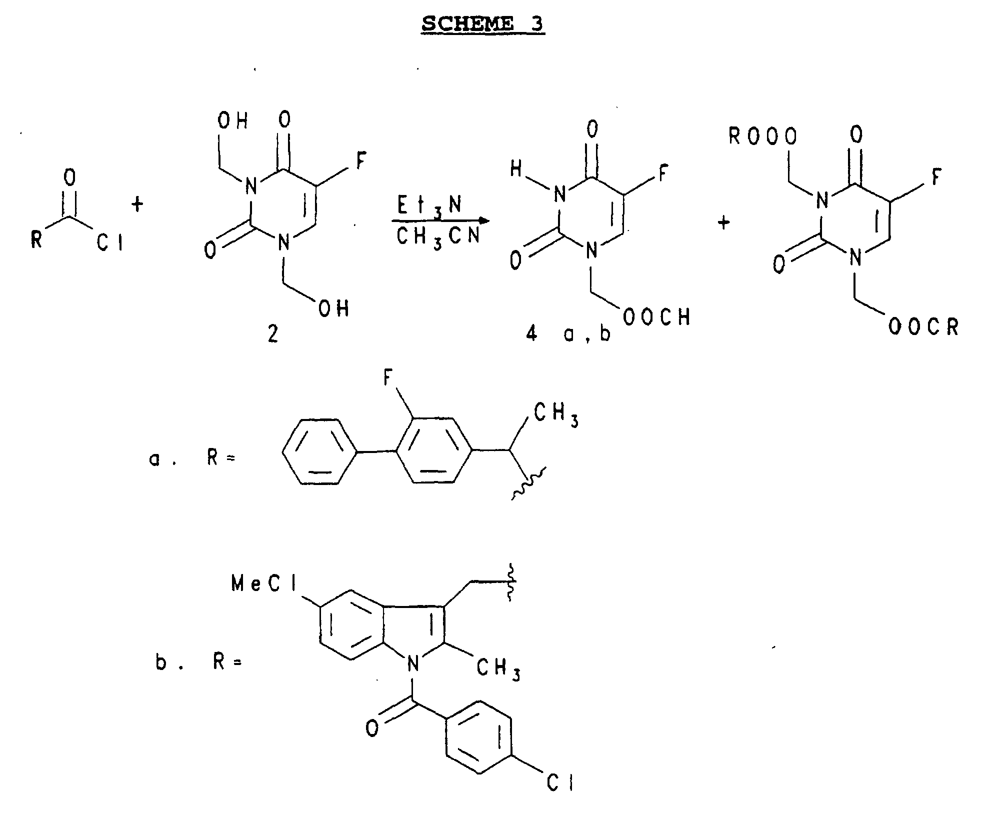 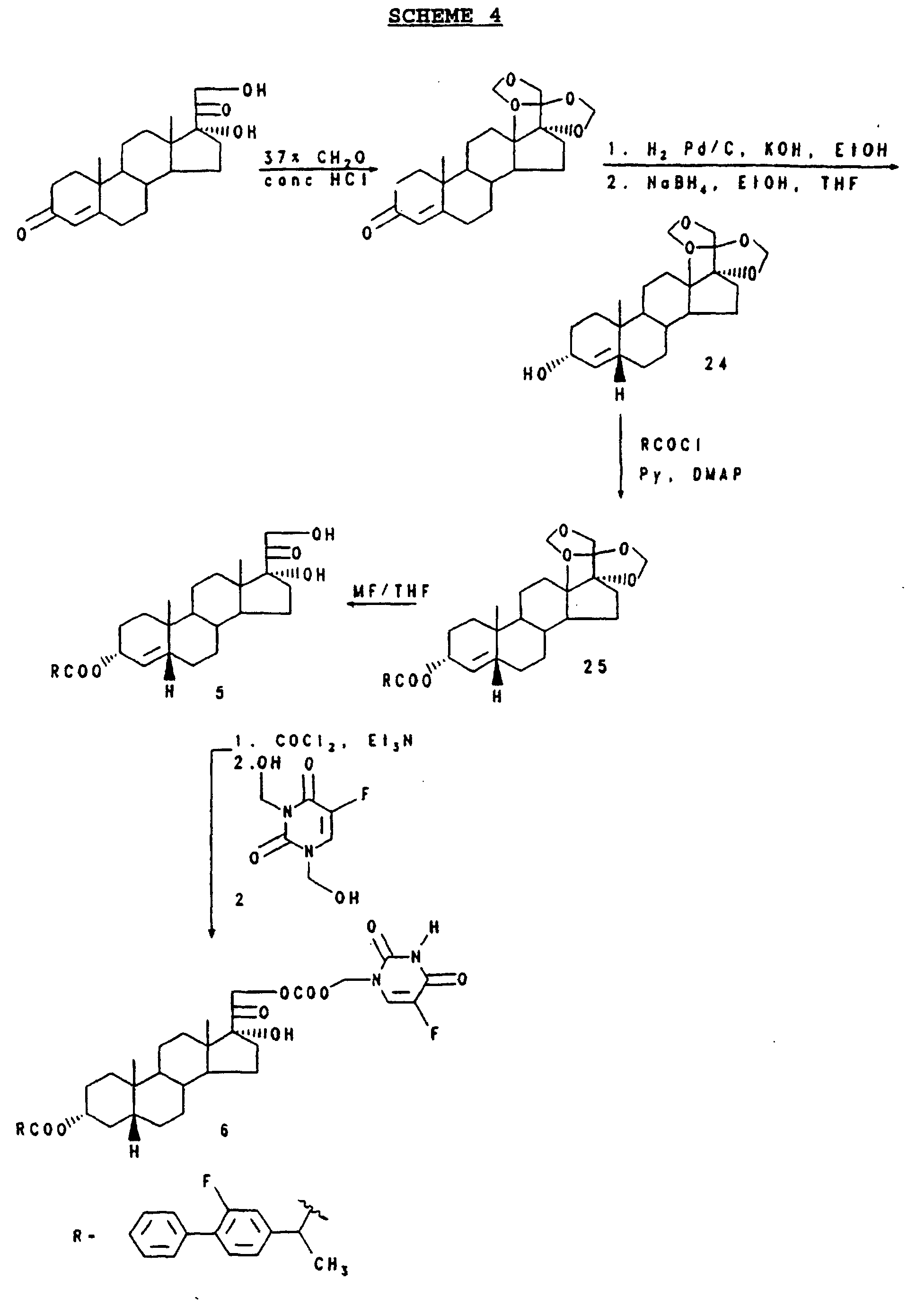 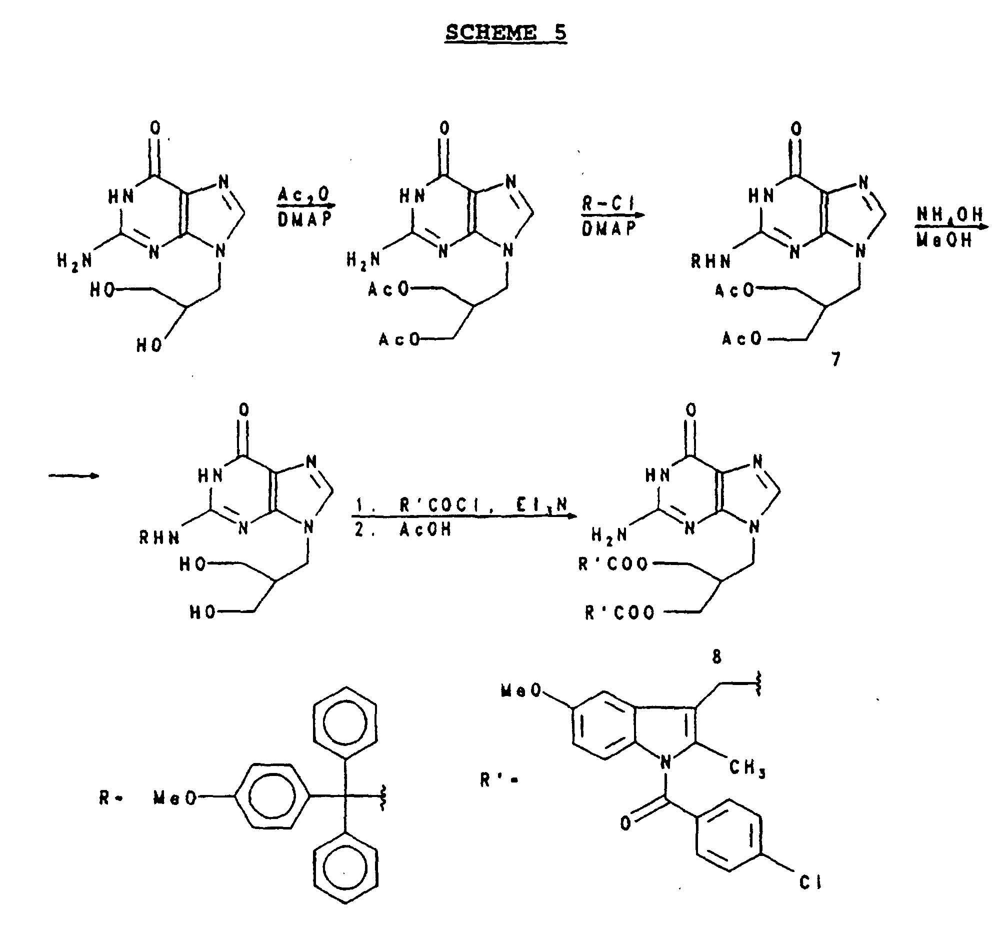 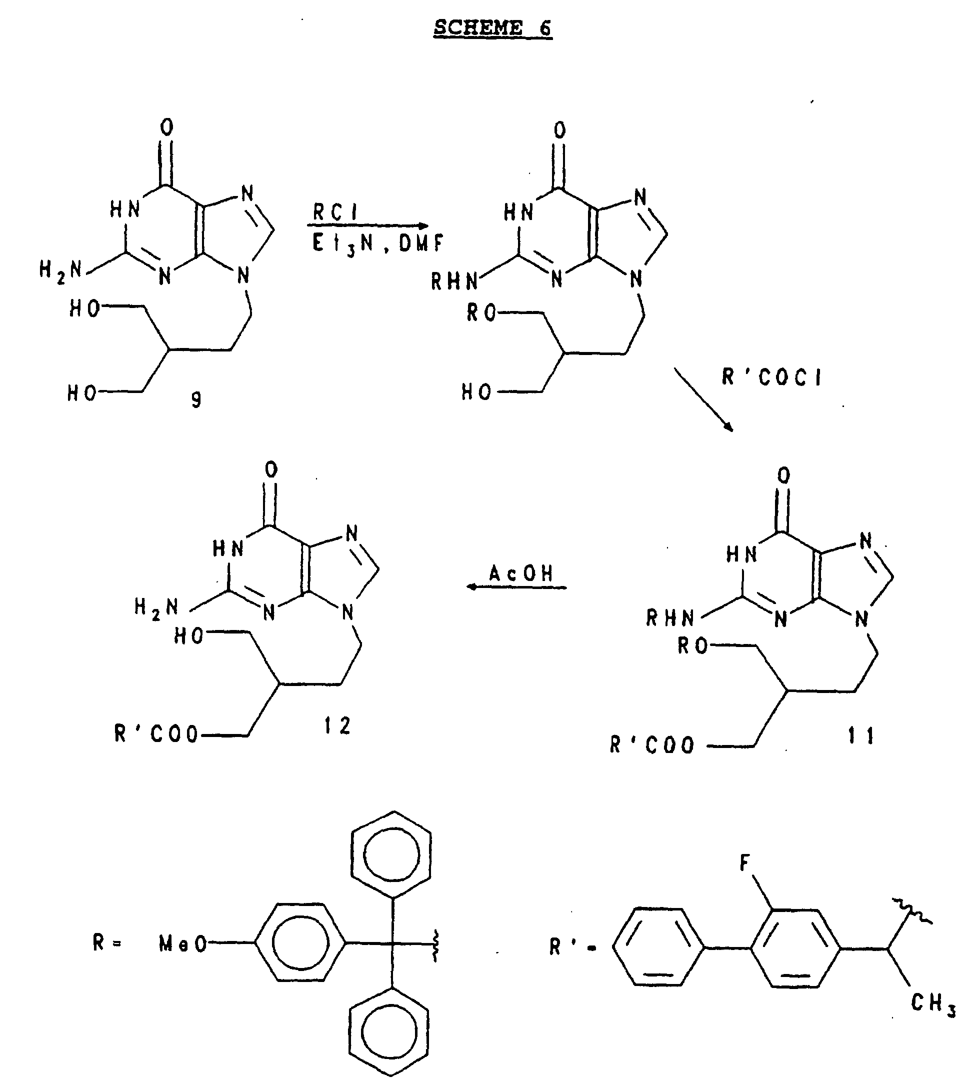 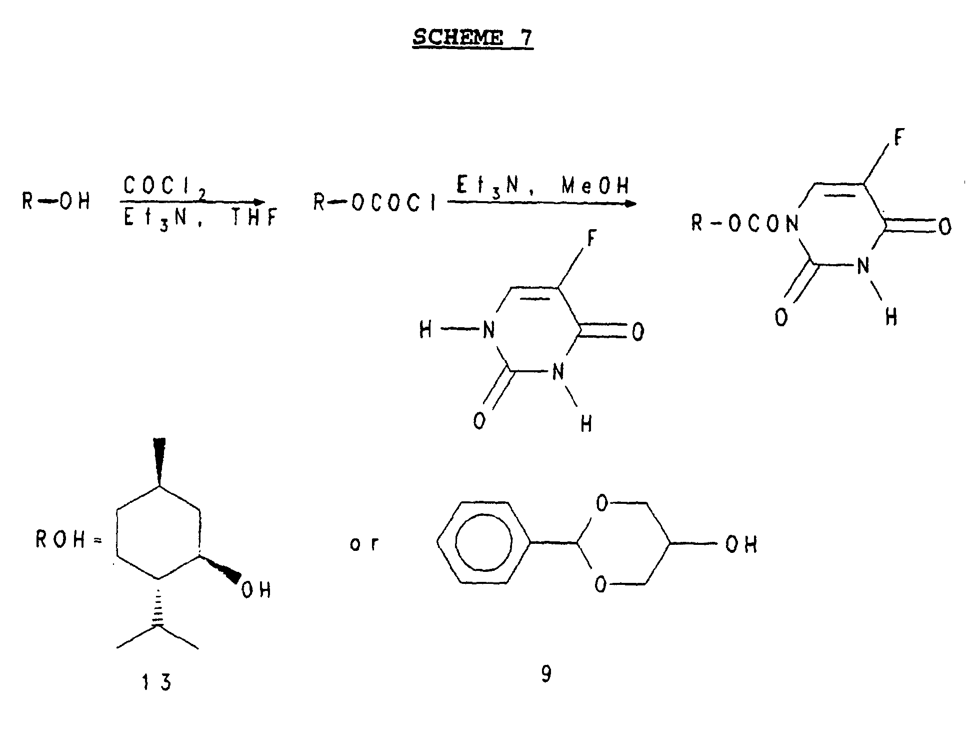 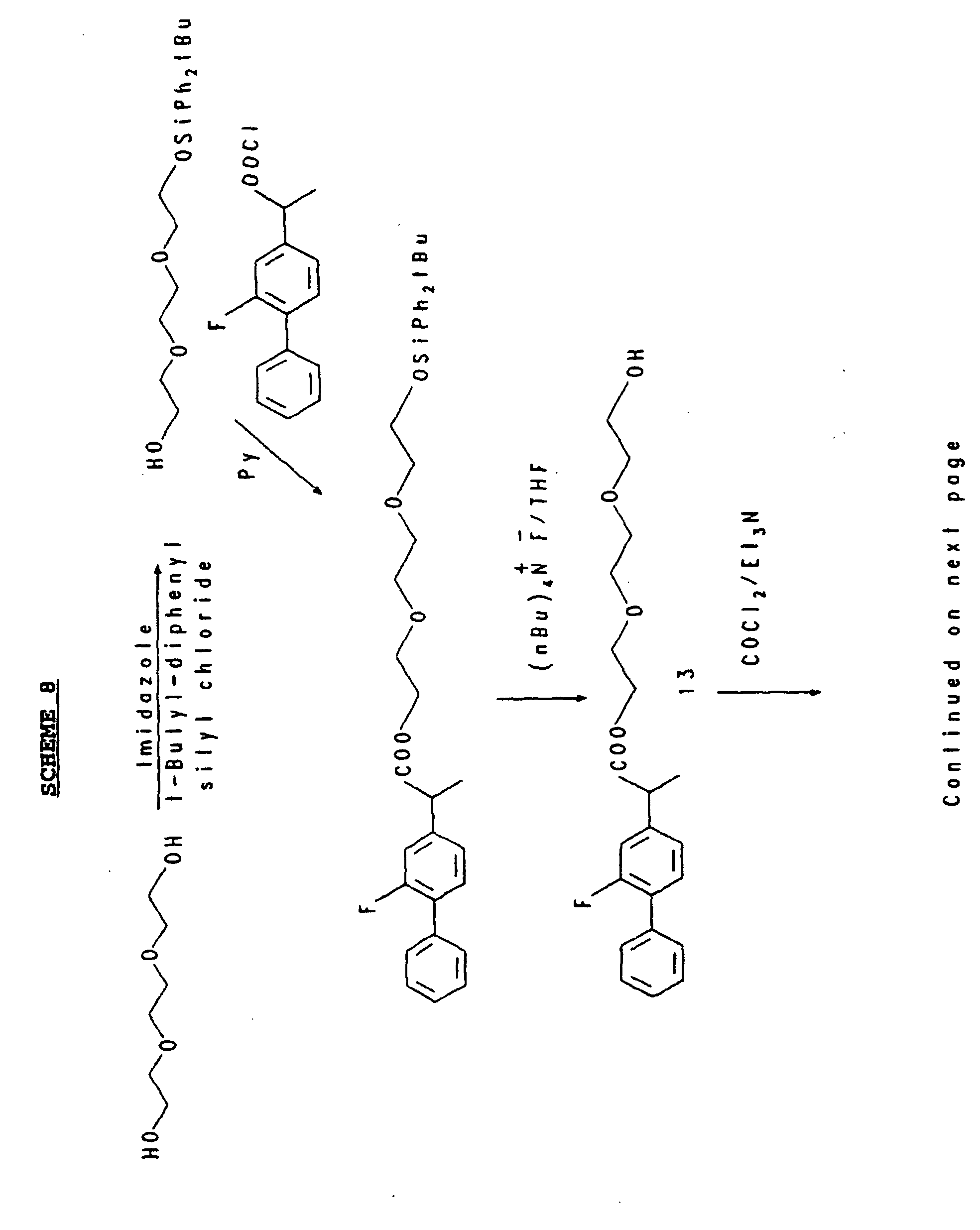 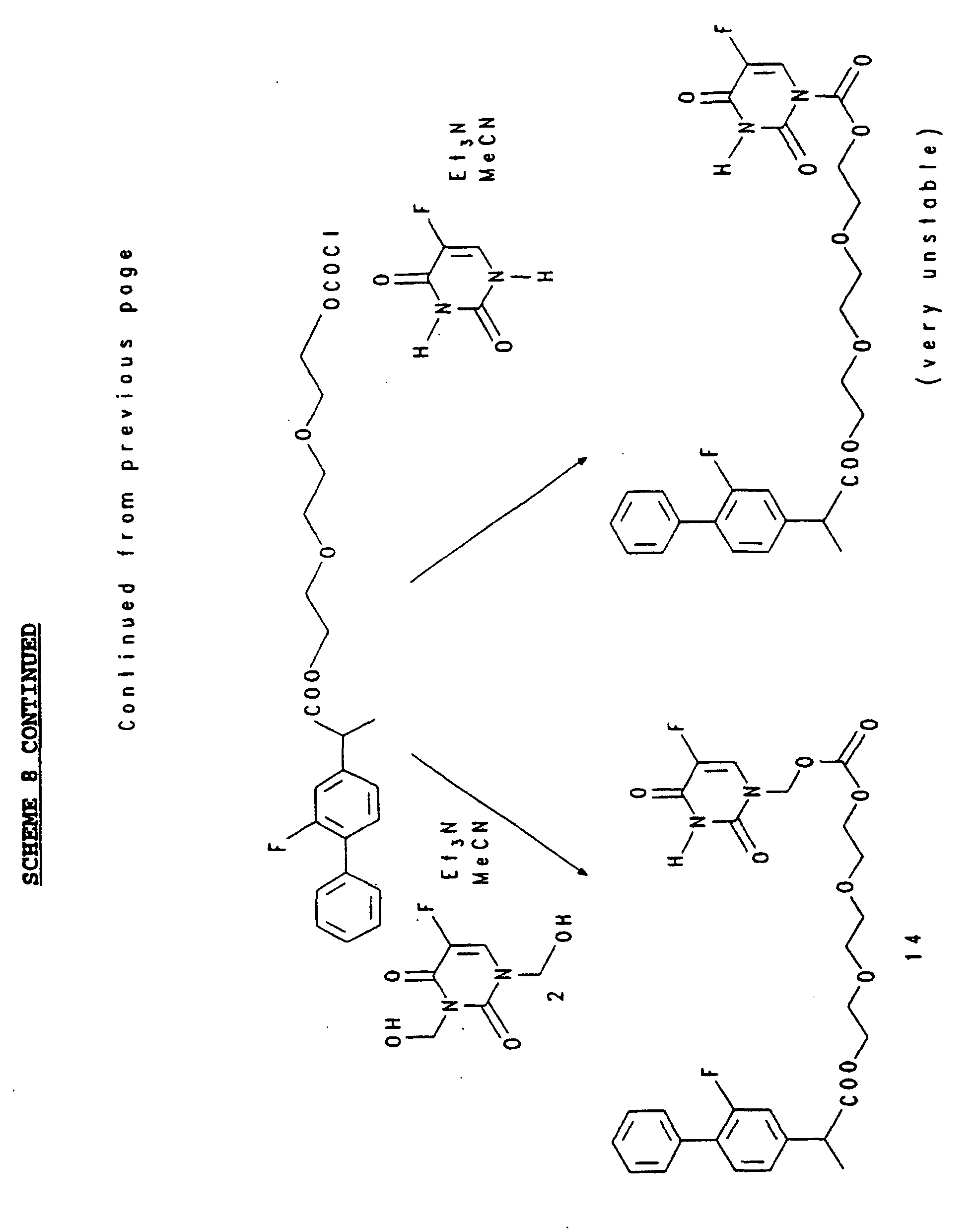 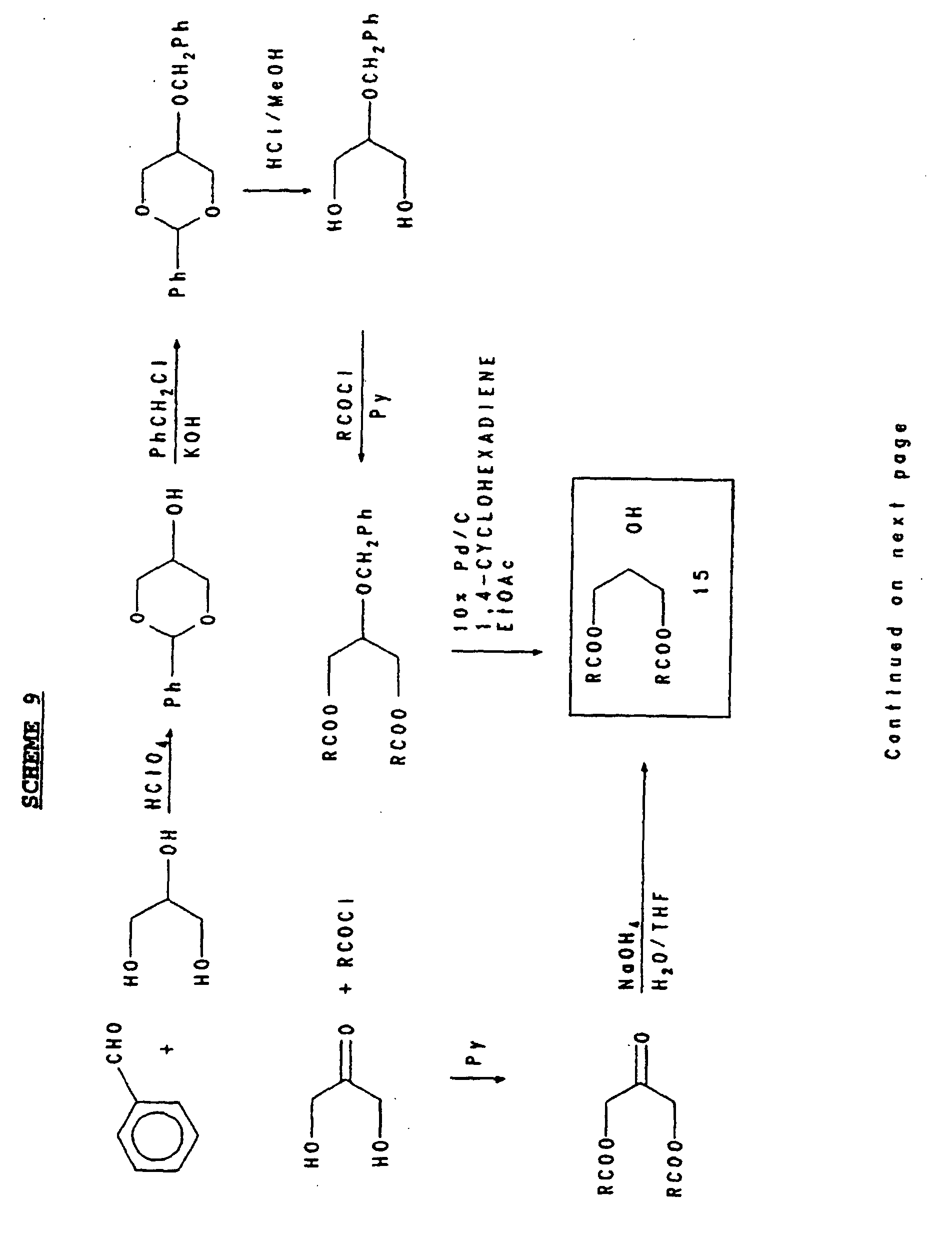 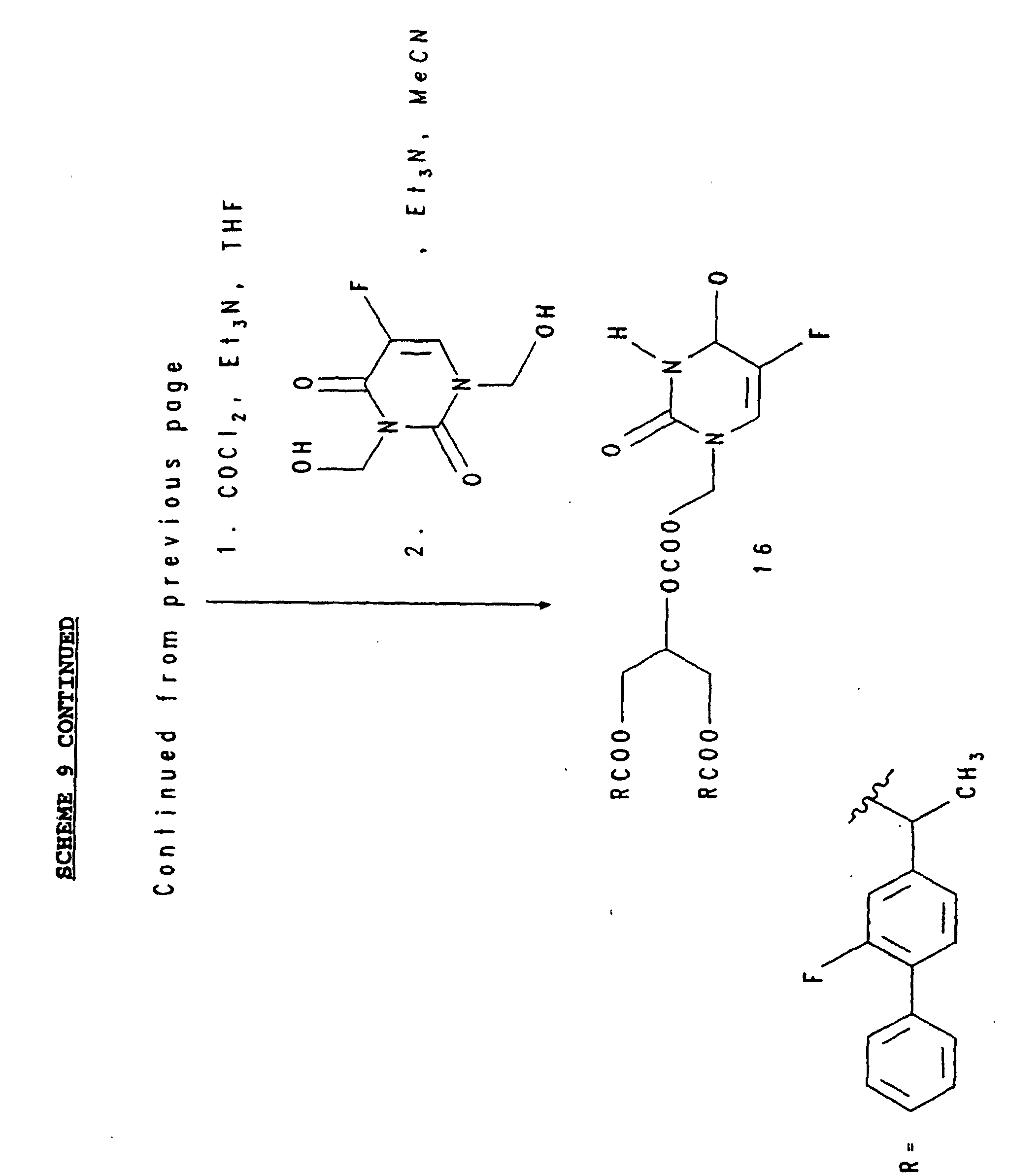 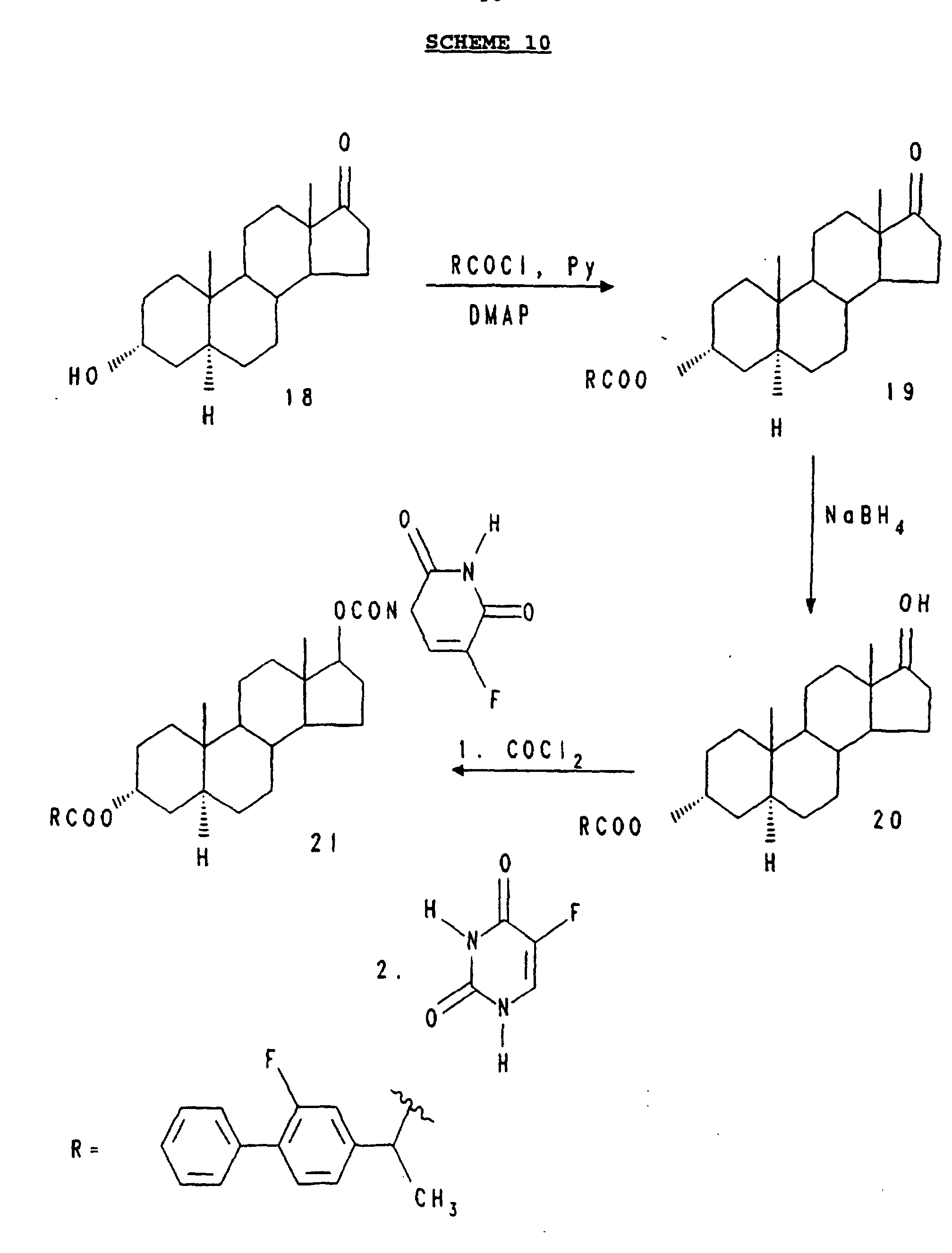 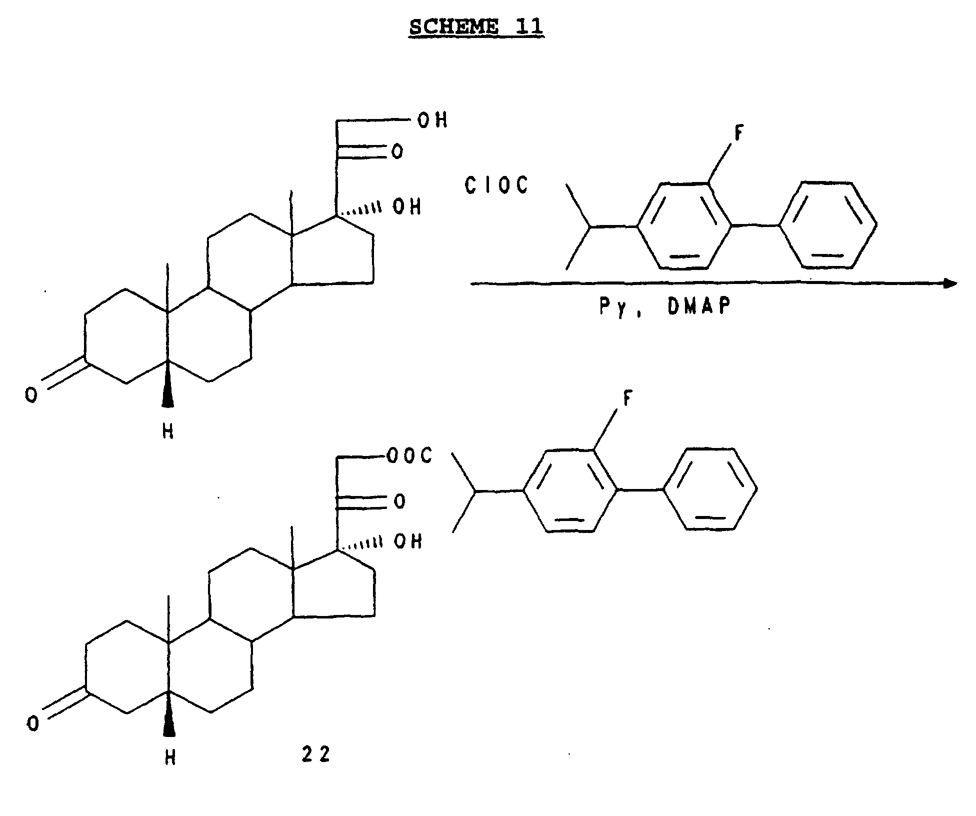 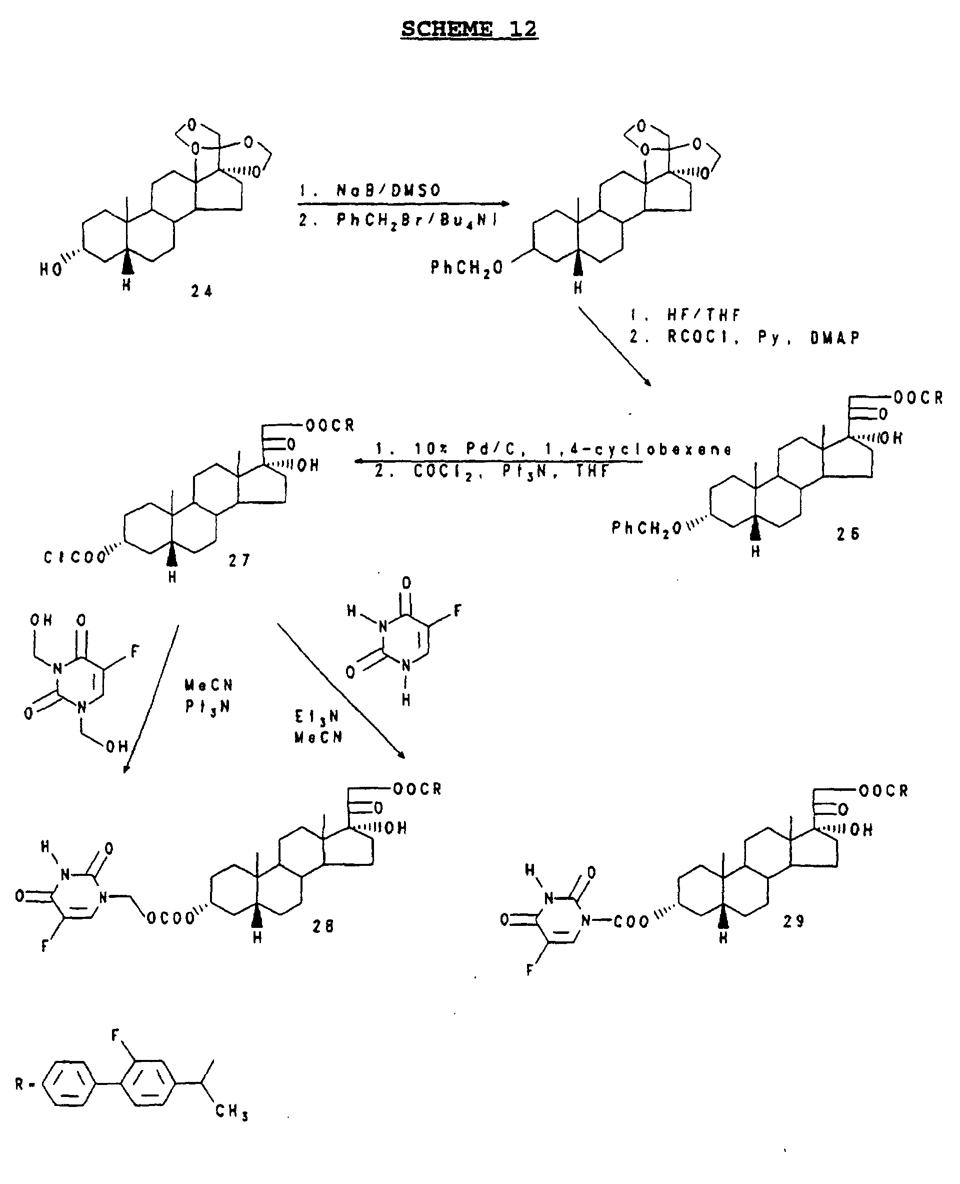 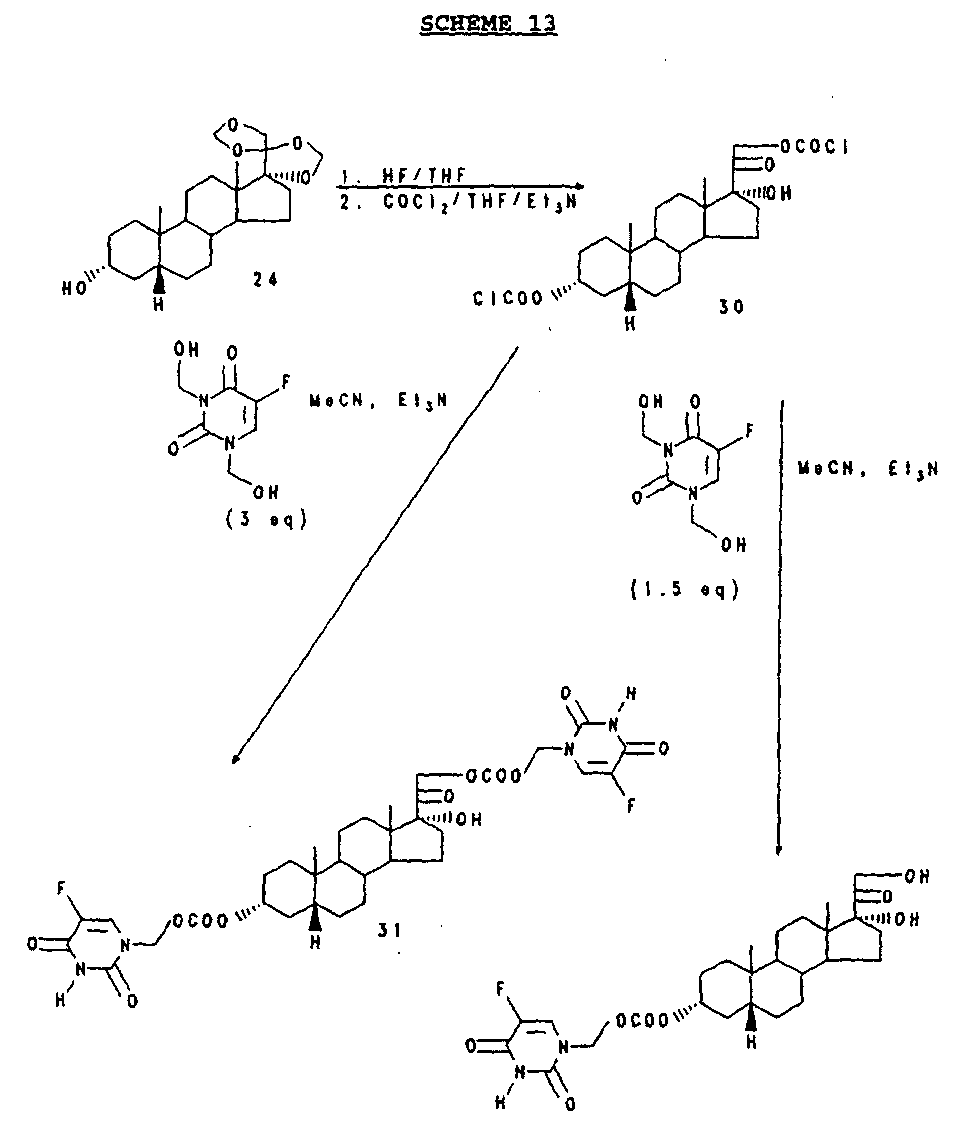 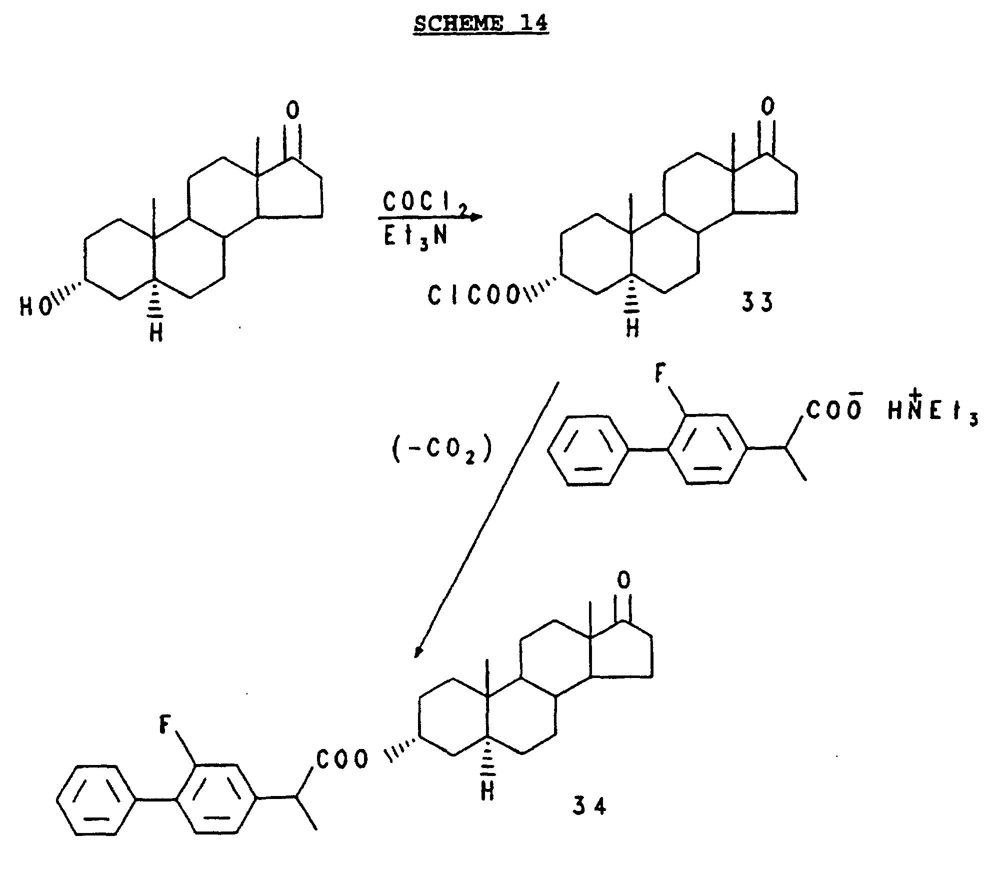 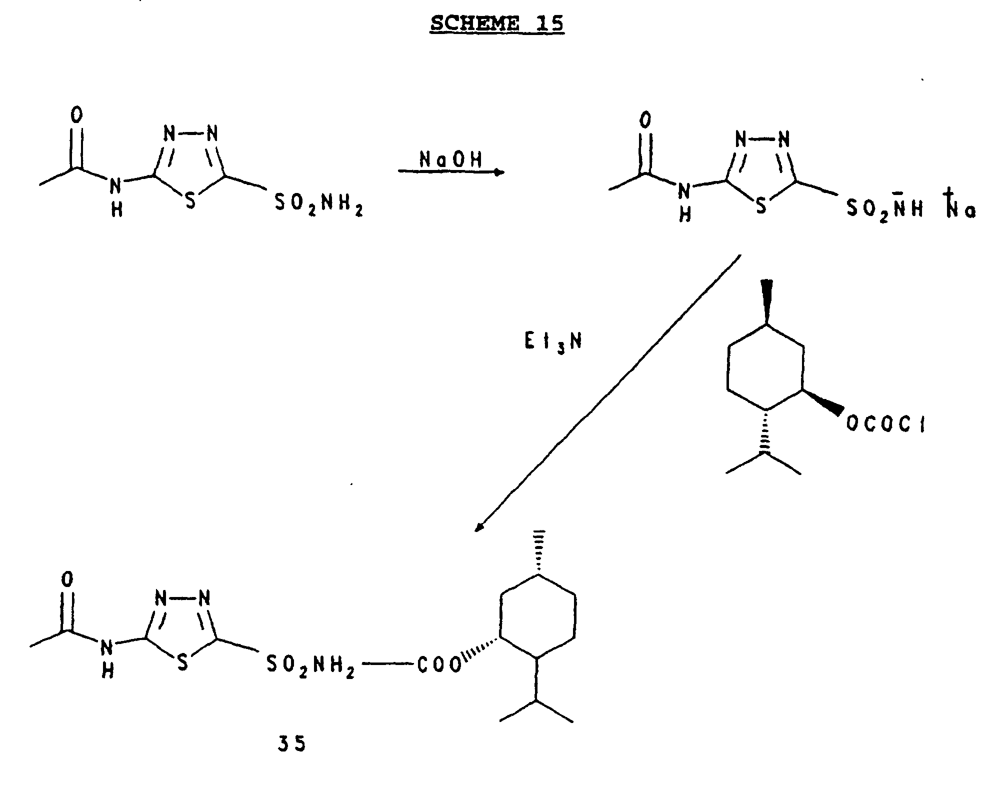 The purpose of the above description and examples is to illustrate some embodiments of the present invention.

## Claims

1. A bioerodible composition that provides sustained release of active drugs in a bodily fluid, comprising a codrug having at least two active drugs covalently linked to one another via a labile bond, wherein the codrug has low solubility in bodily fluids, such that the codrug undergoes slow dissolution into the bodily fluid followed by rapid hydrolysis of the labile bond, thereby regenerating the active drugs.

2. The codrug composition according to claim 1, **characterized in that** at least one of said drug compounds is selected from the group consisting of an anticancer agent, a steroid, an anti-neovascular agent and a nonsteroidal anti-inflammatory agent.

3. The codrug composition according to claim 2, **characterized in that** said anticancer agent is 5-fluorouracil.

4. The codrug composition according to claim 2, **characterized in that** said steroid is triamcinolone acetonide or dexamethasone.

5. The codrug composition according to claim 2, **characterized in that** said anti-neovascular agent is 3α,17α-21-trihydroxy 5β pregnane-20-one (THS).

6. The codrug composition according to claim 2, **characterized in that** said nonsteroidal anti-inflammatory agent is flurbiprofen.

7. The codrug composition according to claim 1, **characterized in that** at least one of said drug compounds is selected from the group consisting of steroid, nonsteroidal anti-inflammatory, anti-cancer, anti-neovaseular drug, prodrug or other therapeutic compound, and further comprising a biological compound with pharmacological activity.

8. The codrug composition according to claim 7, **characterized in that** said biological compound with pharmacological activity is prostaglandin F2 alpha (PGF2α).

9. The codrug composition according to claim 1, charactrized in that it is in a form selected from the group consisting of an injectable form, a solid form and a topical form.

10. The codrug composition according to claim 9, **characterized in that** said injectable form is selected from the group consisting of liposomes, suspensions, microsphere and nanoparticles.

11. The codrug composition according to claim 9, **characterized in that** said topical form is selected from the group consisting of a transdermal patch, ointment, cream, suspension, liquid, elixir and eye drop.

12. The codrug composition according to claim 1, **characterized in that** it is selected from the group consisting of an inhalable composition, an implantable composition, a nasal spray composition, a rectal composition, a vaginal composition and an oral composition.

13. The codrug composition according to claim 1, **characterized by** being fixed to an implantable device or by being a coating on an implantable device.

14. The codrug composition according to claim 13, **characterized in that** said implantable coated device is a suture.

15. The codrug composition according to claim 1, **characterized in that** it is in the form of a nonerodible delivery vehicle or an erodible delivery vehicle.

16. The codrug composition according to claim 15, **characterized in that** said nonerodible delivery vehicle comprises polyvinyl alcohol.

17. The codrug composition according to claim 16, **characterized in that** it comprises from 0.1 to up to about 100% of said nonerodible delivery vehicle.

18. The codrug composition according to claim 15, **characterized in that** it comprises from 0.1 to up to about 100% of said erodible delivery vehicle.

19. The codrug composition according to claim 18, **characterized in that** it further comprises an additional bioerodible substance.

20. The codrug composition according to claim 19, **characterized in that** said additional bioerodible substance is selected from the group consisting of polylactic acid, polyglycolic acid and polalkylcyanoacrylate.

21. The codrug composition according to anyone of claims 18 or 19, **characterized in that** said erodible vehicle further comprises an additional therapeutically active compound.

22. The codrug composition according to claim 21, **characterized in that** said additional therapeutically active compound forms part of the codrug entity.

23. The codrug composition according to claim 22, **characterized in that** said additional therapeutically active compound which forms part of the codrug entity is timolol and said codrug comprises timolol linked to prostaglandin F2 alpha (PGF2α).

24. The codrug composition according to claim 15, **characterized in that** it further comprises a pharmaceutical excipient.

25. The codrug composition according to claim 1, **characterized in that** said codrug compounds are selected from the group consisting of 5FU linked via a carbonate bond to a glycerol diflurbiprofen ester; ganciclovir esterified to pivalic acid and to a succinyl-0-tyrosine ) moiety; cyclic phosphate ester of ganciclovir linked to tyrosine; a cyclic phosphate ester of ganciclovir linked to an alkyl group of another molecule of ganciclovir; conjugation of 5-fluorouracil with a corticosteroid; conjugation of acyclovir with flurbiprofen; conjugation of timolol with the prostaglandin PGF2 alpha, and 3α,17α,21-trihydraxy 5β pregnane-20-one (THS) and 5FU.

26. The codrug composition according to claim 1, **characterized in that** it comprises conjugation of an anti-viral compound selected from the group consisting of ganciclovir and acyclovir with a nonsteroidal anti-inflammatory agent selected from the group ) consisting of flurbiprofen and indomethacin.

27. The codrug composition according to claim 1, **characterized in that** it is in a form selected from the group consisting of a pellet; an injectable bioerodible substance and an implantable bioerodible substance.

28. The codrug composition according to claim 27, **characterized in that** said implantable bioerodible substance is selected from the group consisting of polylactic acid and polyglycol compounds.

29. The codrug composition according to claim 23 for use in the treatment of posterior capsular opacification.

30. The codrug composition according to claim 23 for use in the treatment of herpes keratitis in the form of subconjunctival implantable and bioerodible device.

31. A codrug composition according to claim 1 comprising as the active ingredient 5-fluorouracil(5FU)-triamcinolone (TRI) in the form of an implantable device for use in the prevention and treatment of scar formation occuring after extraocular muscle surgical operations of patients affected by strabismus.

32. A pharmaceutical composition according to claim 1 comprising as active principle a codrug selected from the group consisting of:
- 5FU-TRI or 5FU-DX for the use in the treatment of proliferative vitreoretinopathy;
- 5FU-FB for the use in the treatment of uveitis;
- PGF2α - timolol for use in the treatment of glaucoma;
- 5FU-DX for use in the treatment of cancer;
- 5FU-THS for use in the treatment of neovascularization;
- ganciclovir with flurbiprofen for use in the treatment of cytomegalovirus;
- acyclovir-flurbiprofen for use in the treatment of herpes.

33. Use of a codrug composition according to claim 1 having at least two active drugs covalently linked to one another via a labile bond for the manufacture of a bioerodible medicament for providing sustained release of active drugs in a bodily fluid, wherein the codrug has low solubility in bodily fluids, such that the codrug undergoes slow dissolution into the bodily fluid followed by rapid hydrolysis of the labile bond, thereby regenerating the active drugs.

34. The codrug composition according to claim 1, wherein said codrug comprises an antiproliferative agent and an antiinflammatory agent.

35. The codrug composition according to claim 1, wherein said codrug comprises an anticancer agent and an antiinflammatory agent.

36. The codrug composition according to claim 1, wherein said codrug comprises an antiproliferative agent and a corticosteroid agent.

37. The codrug composition according to claim 1, wherein said codrug comprises an anticancer agent and a corticosteroid agent.

38. The codrug composition according to claim 34 or 36, wherein said antiproliferative agent is 5-fluorouracil.

39. The codrug composition according to claim 35 or 37, wherein said anticancer agent is 5-fluorouracil.

40. The codrug composition according to claim 34 or 36, wherein said antiinflammatory agent is triamcinplone acetonide.

41. The codrug composition according to claim 36 or 37, wherein said corticosteroid agent is triamcinolone acetonide.

42. The codrug composition according to claim 1, wherein said codrug comprises triamcinolone acetonide covalently linked to 5-fluorouracil.

## Patentansprüche

1. Bioerodierbare Zusammensetzung, die anhaltende Freisetzung von Wirkstoffen in einem Körperfluid bereitstellt, umfassend ein Co-Pharmakon mit mindestens zwei Wirkstoffen, die über eine labile Bindung kovalent miteinander verknüpft sind, wobei das Co-Pharmakon geringe Löslichkeit in Körperfluiden hat, so dass das Co-Pharmakon sich langsam in das Körperfluid auflöst, gefolgt von einer schnellen Hydrolyse der labilen Bindung, wodurch die Wirkstoffe regeneriert werden.

2. Co-Pharmakon-Zusammensetzung gemäß Anspruch 1, **dadurch gekennzeichnet, dass** mindestens eine der Arzneimittelverbindungen aus der Gruppe bestehend aus einem krebshemmenden Mittel, einem Steroid, einem Antineovaskularisierungsmittel und einem nicht-steroidalen, entzündungshemmenden Mittel ausgewählt ist.

3. Co-Pharmakon-Zusammensetzung gemäß Anspruch 2, **dadurch gekennzeichnet, dass** das krebshemmende Mittel 5-Fluoruracil ist.

4. Co-Pharmakon-Zusammensetzung gemäß Anspruch 2, **dadurch gekennzeichnet, dass** das Steroid Triamcinolonacetonid oder Dexamethason ist.

5. Co-Pharmakon-Zusammensetzung gemäß Anspruch 2, **dadurch gekennzeichnet, dass** das Antineovaskularisierungsmittel 3α,17α,21-Trihydroxy-5β-pregnan-20-on (THS) ist.

6. Co-Pharmakon-Zusammensetzung gemäß Anspruch 2, **dadurch gekennzeichnet, dass** das nicht-steroidale, entzündungshemmende Mittel Flurbiprofen ist.

7. Co-Pharmakon-Zusammensetzung gemäß Anspruch 1, **dadurch gekennzeichnet, dass** mindestens eine der Arzneimittelverbindungen ausgewählt ist aus der Gruppe bestehend aus einem Steroid, einem nicht-steroidalen, entzündungshemmenden Mittel, einem krebshemmenden Mittel, einem Antineovaskularisierungsmittel, einem Pro-Pharmakon oder einer anderen therapeutischen verbindung und des Weiteren umfassend eine biologische Verbindung mit pharmakologischer Aktivität.

8. Co-Pharmakon-Zusammensetzung gemäß Anspruch 7, **dadurch gekennzeichnet, dass** die biologische Verbindung mit pharmakologischer Aktivität Prostaglandin-F2-alpha (PGF2α) ist.

9. Co-Pharmakon-Zusammensetzung gemäß Anspruch 1, **dadurch gekennzeichnet, dass** sie in einer Form vorliegt, die ausgewählt ist aus der Gruppe bestehend aus einer injizierbaren Form, einer festen Form und einer topischen Form.

10. Co-Pharmakon-Zusammensetzung gemäß Anspruch 9, **dadurch gekennzeichnet, dass** die injizierbare Form ausgewählt ist aus der Gruppe bestehend aus Liposomen, Suspensionen, Mikrokügelchen und Nanopartikeln.

11. Co-Pharmakon-Zusammensetzung gemäß Anspruch 9, **dadurch gekennzeichnet, dass** die topische Form ausgewählt ist aus der Gruppe bestehend aus einem transdermalen Pflaster, einer Salbe, einer Creme, einer Suspension, einer Flüssigkeit, einem Elixir und Augentropfen.

12. Co-Pharmakon-Zusammensetzung gemäß Anspruch 1, **dadurch gekennzeichnet, dass** sie ausgewählt ist aus der Gruppe bestehend aus einer inhalierbaren Zusammensetzung, einer implantierbaren Zusammensetzung, einer Nasenspray-Zusammensetzung, einer rektalen Zusammensetzung, einer vaginalen Zusammensetzung und einer oralen Zusammensetzung.

13. Co-Pharmakon-Zusammensetzung gemäß Anspruch 1, **dadurch gekennzeichnet, dass** sie an einer implantierbaren Vorrichtung befestigt ist oder dass es sich dabei um eine Beschichtung auf einer implantierbaren Vorrichtung handelt.

14. Co-Pharmakon-Zusammensetzung gemäß Anspruch 13, **dadurch gekennzeichnet, dass** es sich bei der implantierbaren, beschichteten Vorrichtung um Nahtmaterial handelt.

15. Co-Pharmakon-Zusammensetzung gemäß Anspruch 1, **dadurch gekennzeichnet, dass** sie in Form eines nicht erodierbaren Transportvehikels oder eines erodierbaren Transportvehikels vorliegt.

16. Co-Pharmakon-Zusammensetzung gemäß Anspruch 15, **dadurch gekennzeichnet, dass** das nicht erodierbare Transportvehikel Polyvinylalkohol umfasst.

17. Co-Pharmakon-Zusammensetzung gemäß Anspruch 16, **dadurch gekennzeichnet, dass** sie zwischen 0,1 bis etwa 100 % des nicht erodierbaren Transportvehikels enthält.

18. Co-Pharmakon-Zusammensetzung gemäß Anspruch 15, **dadurch gekennzeichnet, dass** sie zwischen 0,1 bis etwa 100 % des erodierbaren Transportvehikels enthält.

19. Co-Pharmakon-Zusammensetzung gemäß Anspruch 18, **dadurch gekennzeichnet, dass** sie zusätzlich eine weitere, bioerodierbare Substanz enthält.

20. Co-Pharmakon-Zusammensetzung gemäß Anspruch 19, **dadurch gekennzeichnet, dass** die weitere, bioerodierbare Substanz ausgewählt ist aus der Gruppe bestehend aus Polymilchsäure, Polyglycolsäure und Polyalkylcyanoacrylat.

21. Co-Pharmakon-Zusammensetzung gemäß einem der Ansprüche 18 oder 19, **dadurch gekennzeichnet, dass** das erodierbare Vehikel zusätzlich eine weitere, therapeutisch aktive Verbindung enthält.

22. Co-Pharmakon-Zusammensetzung gemäß Anspruch 21, **dadurch gekennzeichnet, dass** die weitere, therapeutisch aktive Verbindung einen Teil der Co-Pharmakon-Einheit bildet.

23. Co-Pharmakon-Zusammensetzung gemäß Anspruch 22, **dadurch gekennzeichnet, dass** die weitere, therapeutisch aktive Verbindung, die einen Teil der Co-Pharmakon-Einheit bildet, Timolol ist und das Co-Pharmakon Timolol verknüpft mit Prostaglandin-F2-alpha (PGF2α) enthält.

24. Co-Pharmakon-Zusammensetzung gemäß Anspruch 15, **dadurch gekennzeichnet, dass** sie zusätzlich einen pharmazeutischen Trägerstoff enthält.

25. Co-Pharmakon-Zusammensetzung gemäß Anspruch 1, **dadurch gekennzeichnet, dass** die Co-Pharmakon-Verbindungen ausgewählt sind aus der Gruppe bestehend aus 5FU, über eine Carbonatbindung verknüpft mit einem Glycerindiflurbiprofenester; Ganciclovir verestert mit Pivalinsäure und einer Succinyl-O-Tyrosingruppe; einem zyklischen Phosphatester von Ganciclovir verknüpft mit Tyrosin; einem zyklischen Phosphatester von Ganciclovir verknüpft mit einer Alkylgruppe eines anderen Moleküls von Ganciclovir; Konjugation von 5-Fluoruracil mit einem Corticosteroid; Konjugation von Acyclovir mit Flurbiprofen; Konjugation von Timolol mit dem Prostaglandin-PGF2-alpha und 3α,17 α,21 -Trihydroxy-5β-pregnan-20-on (THS) und 5FU.

26. Co-Pharmakon-Zusammensetzung gemäß Anspruch 1, **dadurch gekennzeichnet, dass** sie eine Konjugation einer antiviralen Verbindung ausgewählt aus der Gruppe bestehend aus Ganciclovir und Acyclovir mit einem nicht-steroidalen, entzündungshemmenden Mittel ausgewählt aus der Gruppe bestehend aus Flurbiprofen und Indomethacin umfasst.

27. Co-Pharmakon-Zusammensetzung gemäß Anspruch 1, **dadurch gekennzeichnet, dass** sie in einer Form ausgewählt aus der Gruppe bestehend aus einem Pellet, einer injizierbaren, bioerodierbaren Substanz und einer implantierbaren, bioerodierbaren Substanz vorliegt.

28. Co-Pharmakon-Zusammensetzung gemäß Anspruch 27, **dadurch gekennzeichnet, dass** die implantierbare, bioerodierbare Substanz ausgewählt ist aus der Gruppe bestehend aus Polymilchsäure und Polyglycolverbindungen.

29. Co-Pharmakon-Zusammensetzung gemäß Anspruch 23 zur Verwendung bei der Behandlung eines Nachstars.

30. Co-Pharmakon-Zusammensetzung gemäß Anspruch 23 zur Verwendung bei der Behandlung einer Herpes-Keratitis in Form einer subkonjunktivalen, implantierbaren und bioerodierbaren Vorrichtung.

31. Co-Pharmakon-Zusammensetzung gemäß Anspruch 1, umfassend 5-Fluoruracil(5FU)-triamcinolon (TRI) als den aktiven Inhaltsstoff in Form einer implantierbaren Vorrichtung zur Verwendung bei der Prävention und Behandlung der Bildung von Narben nach chirurgischen Eingriffen an den äußeren Augenmuskeln bei Patienten mit Strabismus.

32. Pharmazeutische Zusammensetzung gemäß Anspruch 1, umfassend als aktives Prinzip ein Co-Pharmakon, ausgewählt aus der Gruppe bestehend aus:
- 5FU-TRI oder 5FU-DX zur Verwendung bei der Behandlung einer proliferativen Vitreoretinopathie;
- 5FU-FB zur Verwendung bei der Behandlung einer Uveitis;
- PGF2α-Timolol zur verwendung bei der Behandlung eines Glaukoms;
- 5FU-DX zur Verwendung bei der Behandlung von Krebs;
- 5FU-THS zur Verwendung bei der Behandlung einer Neovaskularisierung;
- Ganciclovir mit Flurbiprofen zur Verwendung bei der Behandlung einer Zytomegalievirusinfektion;
- Acyclovir-Flurbiprofen zur Verwendung bei der Behandlung von Herpes.

33. Verwendung einer Co-Pharmakon-Zusammensetzung gemäß Anspruch 1 mit mindestens zwei Wirkstoffen, die über eine labile Bindung kovalent miteinander verknüpft sind, zur Herstellung eines bioerodierbaren Medikaments, das anhaltende Freisetzung von Wirkstoffen in einem Körperfluid bereit stellt, wobei das Co-Pharmakon geringe Löslichkeit in Körperfluiden hat, so dass das Co-Pharmakon sich langsam in das Körperfluid auflöst, gefolgt von einer schnellen Hydrolyse der labilen Bindung, wodurch die Wirkstoffe regeneriert werden.

34. Co-Pharmakon-Zusammensetzung gemäß Anspruch 1, wobei das Co-Pharmakon ein proliferationshemmendes Mittel und ein entzündungshemmendes Mittel enthält.

35. Co-Pharmakon-Zusammensetzung gemäß Anspruch 1, wobei das Co-Pharmakon ein krebshemmendes Mittel und ein entzündungshemmendes Mittel enthält.

36. Co-Pharmakon-Zusammensetzung gemäß Anspruch 1, wobei das Co-Pharmakon ein proliferationshemmendes Mittel und ein Corticosteroidmittel enthält.

37. Co-Pharmakon-Zusammensetzung gemäß Anspruch 1, wobei das Co-Pharmakon ein krebshemmendes Mittel und ein Corticosteroidmittel enthält.

38. Co-Pharmakon-Zusammensetzung gemäß Anspruch 34 oder 36, wobei das proliferationshemmende Mittel 5-Fluoruracil ist.

39. Co-Pharmakon-Zusammensetzung gemäß Anspruch 35 oder 37, wobei das krebshemmende Mittel 5-Fluoruracil ist.

40. Co-Pharmakon-Zusammensetzung gemäß Anspruch 34 oder 36, wobei das entzündungshemmende Mittel Triamcinolonacetonid ist.

41. Co-Pharmakon-Zusammensetzung gemäß Anspruch 36 oder 37, wobei das Corticosteroidmittel Triamcinolonacetonid ist.

42. Co-Pharmakon-Zusammensetzung gemäß Anspruch 1, wobei das Co-Pharmakon Triamcinolonacetonid kovalent verknüpft mit 5-Fluoruracil enthält.

## Revendications

1. Composition bioérodable qui permet une libération prolongée de médicaments actifs dans un liquide corporel, comprenant un co-médicament ayant au moins deux médicaments actifs liés de façon covalente l'un à l'autre via une liaison labile, dans laquelle le co-médicament a une faible solubilité dans les liquides corporels, de manière à ce que le co-médicament subisse une lente dissolution dans le liquide corporel suivie d'une rapide hydrolyse de la liaison labile, régénérant ainsi les médicaments actifs.

2. Composition de co-médicaments selon la revendication 1, **caractérisée en ce qu'**au moins l'un desdits composés médicamenteux est choisi dans le groupe constitué d'un agent anti-cancereux, d'un stéroïde, d'un agent anti-néovasculaire et d'un agent anti-inflammatoire non stéroïdien.

3. Composition de co-médicaments selon la revendication 2, **caractérisée en ce que** ledit agent anti-cancereux est le 5-fluoro-uracile.

4. Composition de co-médicaments selon la revendication 2, **caractérisée en ce que** ledit stéroïde est la triamcinolone acétonide ou la dexaméthasone.

5. Composition de co-médicaments selon la revendication 2, **caractérisée en ce que** ledit agent anti-néovasculaire est la 3α,17α-21-trihydroxy 5β prégnane-20-one (THS).

6. Composition de co-médicaments selon la revendication 2, **caractérisée en ce que** ledit agent anti-inflammatoire non stéroïdien est le flurbiprofène.

7. Composition de co-médicaments selon la revendication 1, **caractérisée en ce qu'**au moins l'un desdits composés médicamenteux est choisi dans le groupe constitué d'un stéroïde, d'un agent anti-inflammatoire non stéroïdien, d'un agent anti-cancereux, d'un médicament anti-néovasculaire, d'un pro-médicament ou d'un autre composé thérapeutique, et comprenant en outre un composé biologique ayant une activité pharmacologique.

8. Composition de co-médicaments selon la revendication 7, **caractérisée en ce que** ledit composé biologique ayant une activité pharmacologique est la prostaglandine F2 alpha (PGF2α).

9. Composition de co-médicaments selon la revendication 1, **caractérisée en ce qu'**elle se présente sous une forme choisie dans le groupe constitué d'une forme injectable, d'une forme solide et d'une forme topique.

10. Composition de co-médicaments selon la revendication 9, **caractérisée en ce que** ladite forme injectable est choisie dans le groupe constitué des liposomes, des suspensions, des microsphères et des nanoparticulés.

11. Composition de co-médicaments selon la revendication 9, **caractérisée en ce que** ladite forme topique est choisie dans le groupe constitué d'un timbre transdermique, d'un onguent, d'une crème, d'une suspension, d'un liquide, d'un élixir et de collyre.

12. Composition de co-médicaments selon la revendication 1, **caractérisée en ce qu'**elle est choisie dans le groupe constitué d'une composition inhalable, d'une composition implantable, d'une composition pour pulvérisation nasale, d'une composition rectale, d'une composition vaginale et d'une composition orale.

13. Composition de co-médicaments selon la revendication 1, **caractérisée en ce qu'**elle est fixée à un dispositif implantable ou **en ce qu'**elle est un revêtement sur un dispositif implantable.

14. Composition de co-médicaments selon la revendication 13, **caractérisée en ce que** ledit dispositif implantable revêtu est une suture.

15. Composition de co-médicaments selon la revendication 1, **caractérisée en ce qu'**elle se présente sous la forme d'un véhicule de libération non érodable ou d'un véhicule de libération érodable.

16. Composition de co-médicaments selon la revendication 15, **caractérisée en ce que** ledit véhicule de libération non érodable comprend de l'alcool polyvinylique.

17. Composition de co-médicaments selon la revendication 16, **caractérisée en ce qu'**elle comprend de 0,1 jusqu'à environ 100 % dudit véhicule de libération non érodable.

18. Composition de co-médicaments selon la revendication 15, **caractérisée en ce qu'**elle comprend de 0,1 jusqu'à environ 100 % dudit véhicule de libération érodable.

19. Composition de co-médicaments selon la revendication 18, **caractérisée en ce qu'**elle comprend en outre une substance bioérodable supplémentaire.

20. Composition de co-médicaments selon la revendication 19, **caractérisée en ce que** ladite substance bioérodable supplémentaire est choisie dans le groupe constitué de l'acide polylactique, de l'acide polyglycolique et du polyalkylcyanoacrylate.

21. Composition de co-médicaments selon l'une quelconque des revendications 18 ou 19, **caractérisée en ce que** ledit véhicule érodable comprend en outre un composé thérapeutiquement actif supplémentaire.

22. Composition de co-médicaments selon la revendication 21, **caractérisée en ce que** ledit composé thérapeutiquement actif supplémentaire forme une partie de l'entité co-médicament.

23. Composition de co-médicaments selon la revendication 22, **caractérisée en ce que** ledit composé thérapeutiquement actif supplémentaire qui forme une partie de l'entité co-médicament est le timolol et ledit co-médicament comprend du timolol lié à la prostaglandine F2 alpha (PGF2α).

24. Composition de co-médicaments selon la revendication 15, **caractérisée en ce qu'**elle comprend en outre un excipient pharmaceutique.

25. Composition de co-médicaments selon la revendication 1, **caractérisée en ce que** lesdits composés co-médicamenteux sont choisis dans le groupe constitué du 5FU lié via une liaison carbonate à un diflurbiprofène ester de glycérol : ganciclovir estérifié en acide pivalique et en un groupement succinyl-O-tyrosine ; ester de phosphate cyclique de ganciclovir lié à la tyrosine ; un ester de phosphate cyclique de ganciclovir lié à un groupe alkyle d'une autre molécule de ganciclovir ; conjugaison de 5-fluoro-uracile à un corticostéroïde ; conjugaison d'acyclovir au flurbiprofène ; conjugaison de timolol à la prostaglandine PGF2 alpha, et la 3α,17α-21-trihydroxy 5β prégnane-20-one (THS) et 5FU.

26. Composition de co-médicaments selon la revendication 1, **caractérisée en ce qu'**elle comprend la conjugaison d'un composé anti-viral choisi dans le groupe constitué de ganciclovir et d'acyclovir à un agent anti-inflammatoire non stéroïdien choisi dans le groupe constitué de flurbiprofène et d'indométhacine.

27. Composition de co-médicaments selon la revendication 1, **caractérisée en ce qu'**elle se présente sous une forme choisie dans le groupe constitué d'un granulé, d'une substance bioérodable injectable et d'une substance bioérodable implantable.

28. Composition de co-médicaments selon la revendication 27, **caractérisée en ce que** ladite substance bioérodable implantable est choisie dans le groupe constitué de l'acide polylactique et de composés du polyglycol.

29. Composition de co-médicaments selon la revendication 23 à utiliser dans le traitement de l'opacification capsulaire postérieure.

30. Composition de co-médicaments selon la revendication 23 à utiliser dans le traitement de la kératite herpétique sous la forme d'un dispositif implantable et bioérodable subconjonctival.

31. Composition de co-médicaments selon la revendication 1, comprenant à titre d'ingrédient actif le 5-fluoro-uracile (5FU) - triamcinolone (TRI) sous la forme d'un dispositif implantable à utiliser dans la prévention et le traitement de la formation de cicatrices se produisant après des opérations chirurgicales des muscles extrinsèques de patients atteints de strabisme.

32. Composition pharmaceutique selon la revendication 1, comprenant à titre de principe actif un co-médicament choisi dans le groupe constitué de :
- 5FU-TRI ou 5FU-DX à utiliser dans le traitement de la rétinopathie vitreuse proliférante ;
- 5FU-FB à utiliser dans le traitement de l'uvéite ;
- PGF2α - timolol à utiliser dans le traitement du glaucome ;
- 5FU-DX à utiliser dans le traitement du cancer ;
- 5FU-THS à utiliser dans le traitement de la néovascularisation ;
- ganciclovir avec flurbiprofène à utiliser dans le traitement du cytomégalovirus ;
- acyclovir - flurbiprofène à utiliser dans le traitement de l'herpès.

33. Utilisation d'une composition de co-médicaments selon la revendication 1, comprenant au moins deux médicaments actifs liés de façon covalente l'un à l'autre via une liaison labile pour la fabrication d'un médicament bioérodable permettant une libération prolongée de médicaments actifs dans un liquide corporel, dans laquelle le co-médicament a une faible solubilité dans les liquides corporels, de manière à ce que le co-médicament subisse une lente dissolution dans le liquide corporel suivie d'une rapide hydrolyse de la liaison labile, régénérant ainsi les médicaments actifs.

34. Composition de co-médicaments selon la revendication 1, dans laquelle ledit co-médicament comprend un agent anti-proliférant et un agent anti-inflammatoire.

35. Composition de co-médicaments selon la revendication 1, dans laquelle ledit co-médicament comprend un agent anti-cancereux et un agent anti-inflammatoire.

36. Composition de co-médicaments selon la revendication 1, dans laquelle ledit co-médicament comprend un agent anti-proliférant et un agent corticostéroïdien.

37. Composition de co-médicaments selon la revendication 1, dans laquelle ledit co-médicament comprend un agent anti-cancereux et un agent corticostéroïdien.

38. Composition de co-médicaments selon la revendication 34 ou la revendication 36, dans laquelle ledit agent anti-proliférant est le 5-fluoro-uracile.

39. Composition de co-médicaments selon la revendication 35 ou la revendication 37, dans laquelle ledit agent anti-cancereux est le 5-fluoro-uracile.

40. Composition de co-médicaments selon la revendication 34 ou la revendication 36, dans laquelle ledit agent anti-inflammatoire est la triamcinolone acétonide.

41. Composition de co-médicaments selon la revendication 36 ou la revendication 37, dans laquelle ledit agent corticostéroïdien est la triamcinolone acétonide.

42. Composition de co-médicaments selon la revendication 1, dans laquelle ledit co-médicament comprend de la triamcinolone acétonide liée de façon covalente au 5-fluoro-uracile.
